# FASCICULE DE BREVET EUROPEEN

(11) **EP 4 519 442 B1**
(45) Date de publication et mention de la délivrance du brevet: **08.07.2026**
(21) Numéro de dépôt: 23724801.8
(22) Date de dépôt: 04.05.2023
(51) Int. Cl.: C12N 15/62, C12N 11/16, C12N 9/12, C12N 9/04, C12N 9/06, C12N 9/10, C12N 9/14, C12N 9/88, C12N 9/92, C12N 9/02, C12N 15/52, C12P 7/22, C12P 7/26, C12R 1/19

(54) **PRODUCTION DE MOLECULES PAR CATALYSE ENZYMATIQUE PERIPLASMIQUE**
HERSTELLUNG VON MOLEKÜLEN DURCH PERIPLASMATISCHE ENZYMKATALYSE
PRODUCTION OF MOLECULES BY PERIPLASMIC ENZYMATIC CATALYSIS

(30) Priorité: 06.05.2022 FR 2204333
(43) Date de publication de la demande: 12.03.2025
(73) Titulaire: BIOC3, 31077 Toulouse CEDEX 4 (FR)
(72) Inventeur: LACHAUX, Cléa, 31077 Toulouse CEDEX 04 (FR); GRONDIN, Eric, 31077 Toulouse CEDEX 04 (FR); CAM, Yvan, 31077 Toulouse CEDEX 04 (FR)
(74) Mandataire: Regimbeau
(86) Numéro de dépôt international: PCT/EP2023/061870
(87) Numéro de publication internationale: WO 2023/213978

(56) Documents cités:
- CA-A1- 2 797 786
- FR-A1- 3 118 773
- MO GUO ET AL: "Anchored periplasmic expression (APEx)-based bacterial display for rapid and high-throughput screening of B cell epitopes", BIOTECHNOLOGY LETTERS, vol. 36, no. 3, 5 November 2013 (2013-11-05), Dordrecht, pages 609 - 616, XP055291380, ISSN: 0141-5492, DOI: 10.1007/s10529-013-1400-6
- HARVEY BARRETT R ET AL: "Anchored periplasmic expression, a versatile technology for the isolation of high-affinity antibodies from Escherichia coli-expressed libraries", PROCEEDINGS OF THE NATIONAL ACADEMY OF SCIENCES (PNAS), NATIONAL ACADEMY OF SCIENCES, vol. 101, no. 25, 22 June 2004 (2004-06-22), pages 9193 - 9198, XP002358368, ISSN: 0027-8424, DOI: 10.1073/PNAS.0400187101
- LIANG GUO ET AL: "Enhancement of malate production through engineering of the periplasmic rTCA pathway in Escherichia coli", BIOTECHNOLOGY AND BIOENGINEERING, JOHN WILEY, HOBOKEN, USA, vol. 115, no. 6, 30 March 2018 (2018-03-30), pages 1571 - 1580, XP071052607, ISSN: 0006-3592, DOI: 10.1002/BIT.26580
- GU LEI ET AL: "High-level extracellular production of D-Psicose-3-epimerase with recombinantEscherichia coli by a two-stage glycerol feeding approach", BIOPROCESS AND BIOSYSTEMS ENGINEERING, SPRINGER, DE, vol. 36, no. 11, 14 September 2013 (2013-09-14), pages 1767 - 1777, XP035365292, ISSN: 1615-7591, [retrieved on 20130914], DOI: 10.1007/S00449-013-0952-0
- LEE JAE HYUNG ET AL: "Novel strategy for production of aggregation-prone proteins and lytic enzymes in Escherichia coli based on an anchored periplasmic expression system", JOURNAL OF BIOSCIENCE AND BIOENGINEERING, vol. 116, no. 5, 1 November 2013 (2013-11-01), NL, pages 638 - 643, XP055838593, ISSN: 1389-1723, DOI: 10.1016/j.jbiosc.2013.04.023

## Description

L'invention est telle que définie dans les revendications 1 à 13.

La présente invention appartient au domaine de la production de molécules d'intérêt par des microorganismes génétiquement modifiés, et concerne plus particulièrement l'obtention de composés par catalyse enzymatique conduite dans l'espace périplasmique bactérien.

Elle a pour objet un procédé de production d'un composé d'intérêt faisant intervenir un mélange de bactéries (consortium), modifiées pour exprimer une ou plusieurs enzymes dans leur espace périplasmique telles que définies dans les revendications 1 à 13, qui ensemble catalysent une cascade réactionnelle pour la production d'un composé d'intérêt. L'invention a également pour objet un mélange réactionnel pour la production d'un tel composé. Un kit permettant de préparer ledit milieu, non couvert par l'objet des revendications, est également décrit à titre illustratif.

La production de composés organiques par l'industrie pétrochimique soulève des problèmes cruciaux pour l'avenir en termes de coûts et d'impact environnemental, notamment du fait des grandes quantités de carbone libérées dans l'atmosphère et de l'utilisation de ressources non renouvelables. La production de tels composés par des voix biologiques apparait comme une alternative non polluante et durable. Encore faut-il être en mesure de réaliser les schémas réactionnels adéquats et d'obtenir des quantités suffisantes au regard des applications envisagées, selon des modalités économiquement acceptables. En effet, si certains composés peuvent être obtenus par une simple réaction, d'autres nécessitent des étapes intermédiaires qui complexifient et renchérissent le processus. En outre, de nombreuses réactions font appel à des cofacteurs qu'il convient d'apporter dans le milieu réactionnel en quantité proportionnelle au substrat à transformer, ce qui accroît d'autant les coûts.

Actuellement, différentes technologies mettant en œuvre des procédés biologiques sont utilisées pour la production de molécules. La plus classique est l'extraction de produits d'intérêt, par exemple des arômes ou des molécules thérapeutiques, à partir d'organismes tels que les plantes, par infusion macération, extraction par solvant, ou autre. Toutefois, l'extraction végétale est limitée dans ses applications, car elle présente généralement des rendements assez bas par rapport à la masse traitée.

Elle fait appel à des processus complexes pour isoler et purifier la molécule d'intérêt. Enfin, la disponibilité saisonnière ou géographique de l'organisme source contraint fortement les stratégies d'extraction.

Une autre technique, également bien connue, repose sur l'activité fermentaire de microorganismes, génétiquement modifiés ou non, dont le métabolisme cellulaire produit des molécules d'intérêts, sécrétés dans un milieu de culture. La production d'alcool par des levures en est un exemple parmi les plus anciens. La fermentation présente des avantages certains. Elle peut être réalisée toute l'année à un coût modéré, puisque les réactions sont réalisées par les microorganismes. Les rendements théoriques sont en général intéressants, l'apport de la biologie synthétique et de la biologie des systèmes ayant grandement augmenté l'efficacité de ce type de système. Néanmoins, la fermentation présente des inconvénients notables découlant de la nature vivante du biocatalyseur. Ainsi en pratique, les rendements théoriques sont difficiles à obtenir, la cellule tendant toujours à rediriger les flux engagés pour la production de la molécule d'intérêt vers sa biomasse. Par ailleurs, la molécule d'intérêt est sécrétée dans un milieu de fermentation contenant nécessairement un nombre important d'autres molécules, ce qui complique sa séparation et consomme une grande quantité d'eau qu'il faut ensuite retraiter. En outre, toutes les molécules ne peuvent pas être produites par fermentation, soit du fait de leur toxicité pour la cellule, soit du fait que la molécule est peu ou pas excrétée. S'ajoute à ces difficultés le temps de développement d'un organisme fermentaire industriel, d'une dizaine d'années en moyenne. Ainsi, plusieurs limites de la production par fermentation sont identifiables : i) le couplage de la croissance et de la production limite le rendement maximal de production, ii) l'utilisation de la biologie de synthèse pour ingénierer un micro-organisme et rediriger le flux carboné vers le produit se fait au détriment de la croissance, ce qui impacte la robustesse du procédé, iii) Les conditions opératoires sont limitées aux conditions compatibles avec la croissance microbienne (pH, température, composition du milieu, oxygénation,..), iv) les substrats et produits ne doivent pas présenter de cytotoxicité, la concentration maximale de substrat et de produit sont donc fixées en fonction de leur toxicité, v) le substrat et le produit doivent être capables de traverser la membrane cytoplasmique, et vi) le produit est sécrété dans un milieu de culture chargé d'acides organiques et autre sous-produits, complexifiant la purification du produit et augmentant le coût global du processus.

Plus récemment, des techniques de bioproduction par des cellules entières (dite « whole-cell ») ont permis de lever plusieurs de ces inconvénients. La croissance (phase de production des enzymes) et la production sont découplées, le substrat est uniquement dédié à la synthèse de produit. Les conditions opératoires peuvent être optimisées pour maximiser la production, la compatibilité avec la croissance n'est pas requise. En outre, la purification du produit est simplifiée car le produit est sécrété dans un milieu simple dépourvu des co-produits de croissance. Enfin, si le transfert du substrat/produit à travers la membrane cytoplasmique est limitant, une façon de contourner cette limitation consiste à rendre la paroi cellulaire et les membranes perméables par un traitement chimique (par exemple en ajoutant des détergents ou des solvants) ou physique (par exemple en utilisant un choc thermique). Néanmoins, ces procédures peuvent interférer avec les processus de purification en aval, en plus de causer des dommages cellulaires. En outre la principale limite d'un tel procédé est la nécessité d'ingénierie métabolique pour i) optimiser le niveau d'expression génique du ou des gènes codant pour le(s) enzymes(s) de la voie de production et ii) minimiser les réactions secondaires intracellulaires. Or l'optimisation de la quantité de chaque enzyme dans une cascade enzymatique est cruciale pour la performance globale du procédé. En effet, chaque enzyme d'une voie métabolique est responsable d'une réaction biochimique spécifique nécessaire à la synthèse d'un produit final. Si une enzyme est présente en quantité insuffisante dans la cascade, les substrats en amont peuvent s'accumuler, entraînant une réduction de la vitesse de réaction et, par conséquent, de la production du produit final. En revanche, une quantité excessive d'une enzyme peut également perturber l'équilibre de la cascade enzymatique et réduisant ainsi l'efficacité de l'ensemble du processus. Par conséquent, on comprend l'intérêt d'optimiser la quantité de chaque enzyme afin de maximiser l'efficacité de la réaction globale et la production du produit final.

S'il est possible d'ajuster facilement la concentration d'enzymes purifiées dans le mélange réactionnel, en revanche lorsque ces enzymes sont exprimées *in vivo*, la régulation de leur expression génique est nécessaire pour atteindre le niveau optimal de chaque enzyme.

Actuellement, l'ingénierie métabolique et la biologie synthétique sont utilisées pour améliorer les rendements et les titres des produits. Malgré le développement constant de nouveaux outils d'ingénierie métabolique, il est difficile de prédire quel sera le niveau de production d'une enzyme dans la cellule. La variation du niveau d'expression d'un gène dépend de plusieurs paramètres dont le nombre de copie du gène - déterminé par l'origine de réplication lorsqu'il est porté par un plasmide d'expression, le niveau de transcription déterminé par le choix du promoteur et le niveau traductionnel par le 'Ribosome Binding Site' (RBS). La corrélation entre l'efficacité du système d'expression et le nombre de copies, force de transcription ou de traduction n'est cependant pas linéaire. Une expression élevée peut représenter une charge métabolique cellulaire importante, au détriment de la production et de la croissance. Généralement l'optimisation de l'expression génique relève d'une approche combinatoire entre ces différents éléments afin de déterminer la combinaison optimale de façon empirique.

D'autres techniques enzymatiques encore ont permis de synthétiser des molécules à haute-valeur ajoutée sans recourir à des micro-organismes. Ces techniques dans lesquelles des enzymes purifiées catalysent in vitro des réactions avec une grande spécificité, permettent de contrôler facilement les flux en maitrisant le milieu réactionnel. Les rendements obtenus peuvent alors être proches des rendements théoriques, voire les atteindre. L'utilisation directe d'enzymes, immobilisées ou non, surmonte ainsi certains inconvénients de la fermentation. Cependant, ces enzymes doivent être préalablement produites, purifiées et conditionnées, ce qui rend la technique onéreuse. Même si l'émergence des systèmes dits « cell-free » a pu en partie régler le problème de la purification des protéines (enzymes) en les synthétisant concomitamment dans le milieu où se déroule la réaction catalysée, le coût des cofacteurs souvent indispensables à la réaction reste trop important pour une application de ces techniques à grande échelle. Ainsi, toutes ces approches présentent de sérieux inconvénients. Au demeurant, elles sont limitées à la production de certains composés. Une technique alternative possédant les avantages de la fermentation et ceux de la biocatalyse, mais sans leurs inconvénients respectifs, et pouvant être adaptée à volonté à la synthèse d'une large gamme de molécules d'intérêts, pourrait répondre aux défis économiques et environnementaux actuels.

Dans ce but, la présente invention propose une technologie utilisant la machinerie biologique des cellules microbiennes pour produire différentes enzymes aptes à catalyser les réactions d'un schéma réactionnel conduisant d'un substrat à un composé d'intérêt. Le principe est d'utiliser l'espace périplasmique dont sont pourvues les bactéries (essentiellement les bactéries à Gram négatif) comme espace réactionnel contrôlé, dans lequel une enzyme donnée transforme un substrat et excrète le produit formé dans le milieu de culture. Le produit et les éventuels coproduits excrétés par la première bactérie sont alors disponibles pour servir de substrat à une bactérie exprimant une autre enzyme, différente de l'enzyme produite par la première bactérie, formant ainsi un second produit, et ainsi de suite. On parle ainsi d'une 'cascade de réactions' utilisant un consortium d'au moins 2 bactéries, chaque bactérie exprimant une enzyme différente de l'enzyme produite par l'autre bactérie. D'un point de vue bioprocédé l'accessibilité accrue au substrat offerte par le périplasme est un avantage majeur. L'utilisation de le sécrétion périplasmique des enzymes offre une accessibilité améliorée au substrat, comparée au bioprocédé dit « whole-cell » avec des enzymes cytoplasmiques décrit ci-dessus. L'autre avantage d'exprimer les enzymes dans le périplasme est de pouvoir contrôler le pH et la composition saline du milieu réactionnel. Le pH du périplasme et sa composition ionique sont équivalents à celui du milieu environnant, contrairement au pH et concentrations ioniques du cytoplasme qui sont régulés par le microorganisme afin de maintenir l'homéostasie (Wilks JC et Slonczewski J, 2007). Mais à la connaissance de la Demanderesse, il n'a jamais été décrit jusqu'ici de procédé de bioproduction utilisant un consortium d'au moins deux bactéries exprimant chacune, dans le périplasme, une enzyme différente des enzymes exprimées par les autres bactéries, pour réaliser plusieurs étapes catalytiques en cascade.

En premier lieu, il est apparu de façon surprenante que des bactéries modifiées pour exprimer différentes enzymes de manière périplasmique pouvaient être utilisées dans un même milieu réactionnel, pour réaliser de manière orchestrée, chacune une au moins des différentes réactions d'un schéma réactionnel comportant plusieurs étapes. En effet, selon l'invention, les différentes souches transformées mises en présence dans un même milieu réactionnel, restent opérationnelles et réalisent leur fonction en harmonie.

En second lieu, il a été mis en évidence de manière inattendue, que la paroi périplasmique des bactéries ne faisait pas obstacle à la circulation des produits et réactifs d'une bactérie à l'autre, de sorte que non seulement des schémas comprenant plusieurs réactions sont réalisés en intégralité, et mais aussi que la cinétique réactionnelle est tout à fait satisfaisante pour obtenir le composé désiré en quelques heures seulement.

En outre, et malgré la viscosité du périplasme (état de type gel) lié à la présence de saccharides (Brass et al., 1986), susceptible d'avoir pour effet de ralentir les réactions enzymatiques, contrairement au cytoplasme (état liquide) ou aux solutions aqueuses utilisées *in vitro,* la Demanderesse a observé de façon inattendue que la résistance du milieu n'était pas un frein pour réaliser des cascades réactionnelles selon l'invention.

Enfin, l'on sait qu'un frein majeur à l'utilisation du périplasme pour la bioproduction est l'instabilité des co-facteurs dans ce compartiment, or les co-facteurs sont nécessaires dans la majorité des voies métaboliques. Pour exemple le NAD (NAD+, son état réduit est le NADH) est omniprésent dans les réactions métaboliques, il est le co-facteur de plus de 300 réactions redox (Zhou et al.,2011).

Mais grâce au procédé selon l'invention mettant en œuvre un consortium de micro-organismes, il est possible de recycler les co-facteurs nécessaires aux réactions enzymatiques, chacune des enzymes étant exprimée dans le périplasme de cellules différentes. Les co-facteurs, tels que le NAD(P)H, ne peuvent pas traverser la membrane cytoplasmique des cellules bactériennes, mais ils peuvent diffuser à travers l'espace périplasmique qui sépare la membrane externe de la membrane plasmique des bactéries. Cela permet aux co-facteurs de se déplacer librement entre les différentes cellules du consortium, ce qui leur permet d'être efficacement recyclés et réutilisés pour la production.

Ainsi, des schémas réactionnels séquentiels constituant des voies de biosynthèse complexes, ont pu être élaborés de sorte à être réalisés par plusieurs bactéries exprimant chacune au moins une enzyme dans son périplasme. L'expression périplasmique des enzymes, c'est-à-dire hors de la cellule à proprement parler, offre un milieu réactionnel contrôlé et simplifié, duquel les composés d'intérêt peuvent être extraits sans difficulté. En effet, selon le procédé objet de la présente invention, des enzymes sont produites et transférées dans le périplasme des bactéries, compartiment où elles sont confinées et cependant disponibles et catalytiquement opérationnelles vis-à-vis des composés solubilisés dans le milieu. Dans la mesure où le compartiment périplasmique est aisément accessible aux solutés, les réactions s'y déroulent dans un environnement protégé et beaucoup moins complexe que le cytoplasme cellulaire. Cette externalisation des réactions (hors du cytoplasme cellulaire) évite les problématiques de redirection des flux, de toxicité, de transport, propres à la cellule. Le milieu réactionnel est de fait simplifié et peut être contrôlé pour améliorer les réactions enzymatiques, en particulier par l'apport de cofacteurs et d'activateurs contribuant à une activité optimale. La phase d'extraction finale de la molécule d'intérêt est également facilitée. Sachant que l'extraction peut représenter jusqu'à 70 % du coût de production d'un composé par fermentation, cet aspect constitue un atout essentiel.

Un aspect caractéristique de la technique proposée est que le processus de production de la biomasse est découplé de celui de la production de la molécule d'intérêt. La phase de culture cellulaire et la phase de synthèse biochimique se déroulent dans des réacteurs distincts, avec des milieux différents. De ce fait, la biomasse se trouvant dans le réacteur peut être utilisée pour un nouveau processus de synthèse, ou être récupérée à l'issue de la synthèse d'un composé et réutilisée pour un cycle de synthèse ultérieur. Il est alors possible de réaliser de multiples réactions à partir d'une même biomasse. Les coûts de production de la biomasse (multiplication cellulaire des différentes souches) en sont réduits d'autant, notamment du fait des volumes d'eau sensiblement moindres à retraiter. Ce faisant, les coûts de production restent sensiblement ceux des coûts de production microbiens.Un autre avantage notable de la dissociation des processus de production de biomasse et de synthèse de composés est qu'on évite la compétition dans l'emploi des sources de carbone. Dans un procédé classique de fermentation, on cherche à maximiser l'utilisation du carbone pour la production du composé d'intérêt afin d'atteindre un rendement élevé, mais cette optimisation risque de nuire à la croissance cellulaire (production de biomasse), et d'handicaper aussi par contrecoup la production du composé d'intérêt. La balance entre croissance cellulaire et production du composé d'intérêt est donc critique. Au contraire, dans le procédé inventif, la production de biomasse étant découplée de la production de molécule, les sources de carbone sont distinctes. On peut donc optimiser séparément, à la fois la croissance de la biomasse et le rendement de production du composé d'intérêt. En outre, la Demanderesse a montré qu'il était possible d'augmenter considérablement la production d'un produit d'intérêt en modulant les proportions de chaque biocatalyseur (1 bactérie exprimant 1 enzyme) dans le consortium. C'est un avantage considérable lié au système modulaire du procédé de l'invention, comparativement aux procédés précédemment décrits qui nécessiteraient des modifications génétiques complexes d'une même bactérie ou d'autres leviers pour augmenter la production. En particulier, on sait que l'efficacité des peptides signal dépend de la protéine cible à produire. Par exemple, un peptide signal qui favorise une synthèse et une sécrétion de protéines à haut niveau pour une protéine recombinante donnera souvent une synthèse et une sécrétion de protéines à faible niveau pour une autre (Karyolaimos et al., 2019). Étant donné qu'il n'est pas possible de prédire quel sera la performance d'un peptide signal pour une protéine recombinante donnée, il est nécessaire de cribler les bibliothèques de peptides signal pour en trouver un qui favorise une synthèse et une sécrétion de protéines à haut niveau. Cette approche est à la fois chronophage et coûteuse. Grâce au procédé selon l'invention, il est possible de moduler la quantité de chaque biocatalyseur (ex : augmenter la quantité du biocatalyseur pour pallier la faible efficacité du peptide signal) dans le mélange réactionnel de production d'un produit pour maximiser l'efficacité de la réaction. On peut ainsi utiliser un seul signal peptide (ex : pelB), quelle que soit la protéine à produire, et augmenter la proportion du biocatalyseur dans le cas où le signal peptide n'a pas l'efficacité attendue sur la production de la protéine. Cette stratégie permet d'améliorer facilement la production d'un produit, ce qui est avantageux pour une utilisation industrielle. Le procédé objet de l'invention qui va être décrite en détail ci-après, peut être qualifié de système modulaire, dans la mesure où différentes bactéries, chacune transformée pour exprimer une enzyme dans son périplasme, différente des enzymes exprimées par les autres bactéries du système, peuvent être cultivées et conservées, de manière à fournir à volonté les effecteurs d'une large gamme de réactions dans laquelle on peut puiser pour construire un schéma réactionnel complet. On parlera d'une bibliothèque de biocatalyseurs, chacun comprenant une bactérie exprimant dans son périplasme une enzyme, différente des enzymes exprimées par les autres bactéries de la bibliothèque de biocatalyseurs. Les bactéries choisies pour intervenir dans un schéma défini vont être introduites dans un unique milieu pour agir de concert afin de générer un produit d'intérêt. Cette démarche reposant sur la mise en œuvre combinée de modules cellulaires, s'est avérée adaptée à l'obtention d'une grande variété de composés, conférant au procédé inventif un caractère universel inédit. En particulier, des molécules à haute valeur ajoutée, rares ou difficiles d'accès (peu excrétées, toxiques, racémiques d'isomères difficiles à séparer, ...), ont pu être obtenues avec des rendements élevés et un bilan économique et environnemental particulièrement favorable.

L'utilisation de consortium microbiens synthétiques selon le procédé de l'invention présente ainsi de nombreux avantages par rapport aux procédés de bioproduction de l'art antérieur : i) surmonter la charge métabolique élevée portée classiquement par une unique souche en divisant la charge et permettre d'optimiser les voies de manière modulaire, ii) permettre une flexibilité dans l'équilibrage des flux métaboliques entre les modules individuels en contrôlant facilement le rapport des souches modifiées, iii) empêcher l'inhibition potentielle exercée par les intermédiaires métaboliques sur les enzymes sensibles, iv) enfin grâce à sa nature modulaire, permettre la production facile d'une variété de produits chimiques en mélangeant simplement différentes souches microbiennes. Le procédé selon l'invention présente ainsi l'avantage d'éviter les problèmes liés à la co-expression de plusieurs enzymes dans une seule cellule et de s'affranchir de la complexité de la régulation de l'expression génique. De plus, les quantités de chacune des espèces de bactéries modifiées peuvent être optimisées pour une production plus efficace du produit d'intérêt.

Plus précisément, la présente invention a pour objet un procédé de production d'un composé d'intérêt à partir d'un substrat organique, qui comprend les étapes consistant à :
a) - préparer un mélange réactionnel comprenant, dans un milieu convenable, qui est un milieu aqueux comme décrit ci-après dans la description,
   - n bactéries, n étant un nombre entier au moins égal à 2, chaque bactérie étant génétiquement modifiée pour exprimer dans son espace périplasmique une seule enzyme E1, E2, ..., En, chacune desdites n bactéries exprimant une enzyme différente de celle exprimée dans les autres bactéries,
      - ladite enzyme E1 étant apte à catalyser une première réaction R1 à partir dudit substrat organique pour fournir un premier produit P1, et optionnellement un premier coproduit CoP1,

   - chacune desdites enzyme E2, ..., En, étant apte à catalyser une réaction R2, ..., Rn, à partir d'un produit ou d'un coproduit obtenu par une réaction Rn-1, pour fournir respectivement un produit P2, ..., Pn, et optionnellement un coproduit CoP2, ..., CoPn,
   - ledit substrat organique, et
   - optionnellement, un cofacteur CoF1 dudit substrat organique, un cofacteur CoFn desdits produits obtenus par une réaction Rn-1; et/ou un substrat complémentaire SC d'un coproduit obtenu par une réaction Rn-1,
b) - laisser réagir le mélange réactionnel ainsi obtenu, et
c) - séparer la biomasse du surnageant et extraire de celui-ci ledit composé d'intérêt constitué par l'un des produits P1, P2, ..., Pn.

Selon l'invention revendiquée, chaque bactérie est génétiquement modifiée pour exprimer dans son espace périplasmique une seule enzyme E1, E2, ..., En, chacune desdites n bactéries exprimant une enzyme différente de celle exprimée dans les autres bactéries.

Le procédé inventif met en œuvre au moins deux bactéries génétiquement modifiées, chacune étant apte à sécréter une enzyme donnée, différente de l'enzyme exprimée par l'autre bactérie. En fonction du schéma réactionnel élaboré pour produire le composé souhaité, il peut comprendre plusieurs bactéries, par exemple plus de dix, et même plusieurs dizaines. Ce nombre découle du nombre d'enzymes nécessaires à l'obtention d'un produit recherché à partir d'un substrat donné (lui-même découlant du nombre d'étapes réactionnelles), qui va décider du nombre de modules cellulaires à utiliser, sans forcément être égal. Selon l'invention, chaque bactérie exprimera une seule enzyme, différente des enzymes exprimées par les autres bactéries. On pourra notamment utiliser de 2 à 20 bactéries, notamment de 3 à 15, voire de 4 à 12, chaque bactérie exprimant une enzyme différente des enzymes exprimées par les autres bactéries.

Chaque bactérie est chargée de la production d'une seule enzyme particulière, de sorte que si n enzymes doivent intervenir dans le schéma réactionnel retenu, n bactéries seront impliquées dans le procédé. L'expression "module" ou "module cellulaire" pourra être employée pour désigner une bactérie génétiquement transformée et une des enzymes R1, R2, ..., Rn spécifiquement exprimée par celle-ci. On précise que les entiers de 1 à n peuvent donner une indication de l'ordre d'intervention de chaque enzyme dans le schéma réactionnel choisi, sans toutefois que cela soit une règle systématique, certains schémas réactionnels étant linéaires, mais d'autres comportant des ramifications. C'est le cas en particulier lorsqu'un module sert à régénérer un cofacteur, ce qui sera décrit en détail plus loin. Quoi qu'il en soit, des indices n et n-1, ou n et n+1 s'appliquent à des réactions directement consécutives l'une en aval ou en amont de l'autre, ainsi qu'aux enzymes et composés mis en jeu dans lesdites réactions.

Ainsi, une réaction Rn-1 est définie comme étant directement en amont d'une réaction Rn dans le schéma réactionnel, de sorte que le produit Pn-1 et le coproduit CoPn-1 s'il existe, issus de la réaction Rn-1 sont présents dans le milieu et disponibles pour servir respectivement, de substrat, ou le cas échéant de cofacteur, à la réaction Rn directement en aval. Ce principe général souffre une exception concernant la première réaction du schéma réactionnel, dans la mesure où celle-ci est obligatoirement réalisée à partir du substrat organique choisi qui est transformé par l'action d'une première enzyme R1 pour donner un premier produit P1, et le cas échéant un premier coproduit CoP1. A chaque étape, un produit P1, P2, ..., Pn est formé, ainsi qu'optionnellement un coproduit CoP1, CoP2, ..., CoPn.

Le mélange réactionnel préparé à la première étape du procédé selon l'invention comprend, outre les modules cellulaires matérialisant le schéma réactionnel défini, une quantité du substrat organique constituant la matière première à transformer pour obtenir in fine le composé d'intérêt désiré. Ce substrat organique initial va subir une première réaction catalysée par l'enzyme du premier module. Ensuite, lors de chaque étape réactionnelle suivante, le produit synthétisé lors de la réaction juste précédente, pourra jouer le rôle de substrat pour donner un nouveau produit, et ainsi de suite. Dans ce schéma réactionnel linéaire, le produit obtenu en fin de compte sera le composé d'intérêt désiré.

Des cofacteurs peuvent être requis au moins pour certaines des réactions, qui vont donner les coproduits correspondants. Le terme "coproduit" désigne ici spécifiquement une espèce chimique formée à partir d'un cofacteur dans la réaction enzymatique concernée. A noter que ces coproduits sont généralement à même de faire fonction de cofacteur dans d'autres réactions. Ainsi, il pourra être nécessaire d'introduire dans le mélange réactionnel le cas échéant, un cofacteur du substrat organique pour la première réaction, ou un cofacteur du produit obtenu par une réaction Rn-1 qui va jouer le rôle de substrat dans la réaction R suivante.

Lorsque dans une réaction Rn-1, un substrat Sn-1 et un cofacteur CoFn-1 réagissent, un coproduit CoPn-1 est formé aux côtés d'un produit Pn-1. Un schéma réactionnel peut prévoir qu'un coproduit CoPn-1 soit lui-même consommé en tant que cofacteur CoFn dans la réaction Rn suivante, aux côtés d'un substrat complémentaire. Il est alors particulièrement avantageux que la réaction Rn soit à même de reformer un cofacteur CoFn-1. De la sorte, on peut régénérer le premier cofacteur, ce qui dispense d'en faire un apport massif dans le mélange réactionnel. Il convient toutefois d'apporter ledit substrat complémentaire en quantité suffisante dans le mélange réactionnel. Dans la présente description, l'expression "substrat complémentaire" désigne un composé différent du substrat organique initial, qui est utilisé pour réagir en tant que substrat aux côtés d'un cofacteur. Dans ce cas de figure, il sera nécessaire d'introduire dans le mélange réactionnel un substrat complémentaire du cofacteur concerné, en l'occurrence du coproduit CoPn-1, qui vont réagir ensemble dans la réaction R suivante.

En résumé, on comprend que selon le schéma réactionnel défini, un ou plusieurs cofacteurs peuvent intervenir, de même qu'un ou plusieurs substrats complémentaires. C'est pourquoi, à l'étape a) du procédé selon l'invention, le cas échéant, les différents cofacteurs et substrats complémentaires sont ajoutés dans le mélange réactionnel.

Le substrat organique peut être un composé organique quelconque, la seule réserve étant qu'il est susceptible de constituer un substrat dans une réaction enzymatique. Il peut donc être de nature très diverse, par exemple un hydrocarbure linéaire ou ramifié et possiblement substitué, tel qu'une amine, un acide, un hydrate de carbone, un acide aminé, ou autre.

A l'étape a) du procédé inventif, les modules cellulaires et le substrat organique, avec éventuellement les cofacteurs et/ou substrats complémentaires impliqués, sont introduits dans un milieu convenable, c'est-à-dire un milieu qu'un homme du métier peut préparer sans difficulté selon les règles de l'art qu'il maîtrise. Un tel milieu est un milieu aqueux, généralement additionné de minéraux et d'un tampon, comme ceux donnés plus loin en exemple. On le distinguera du milieu réactionnel à proprement parler qui est quant à lui défini comme la phase aqueuse dans laquelle les réactions du schéma réactionnel se déroulent : ceci englobe le milieu dans lequel les bactéries sont plongées ainsi que leur espace périplasmique, puisque les solutés circulent aisément de l'un à l'autre à travers la paroi périplasmique externe. Un tampon simple est utilisé pour faciliter la purification finale du composé obtenu.

Il est précisé que de déroulé des étapes a) et b) peut ne pas être strictement successif. En effet, le procédé peut être conduit en mode continu ou semi-continu, avec apport permanent ou séquentiel de substrat et de réactifs. En outre, dans certains cas, il est préférable de ne pas ajouter dès le départ un substrat complémentaire et/ou un cofacteur dans le mélange réactionnel, mais de le faire dans un certain délai après l'initialisation du processus réactionnel. De même, un ou plusieurs modules cellulaires peuvent être introduits de manière différée. En d'autres termes, selon cette modalité particulière, une action de l'étape a) du procédé se déroule alors que l'étape b) a débuté. Cette chronologie est incluse dans le cadre de la présente invention.

On comprend de ce qui précède que le procédé objet de la présente invention offre de multiples possibilités pour concevoir des schémas réactionnels adaptés à la synthèse de composés d'intérêt variés. Il peut se décliner de différentes manières qui vont maintenant être présentées en détail.

Comme déjà exposé, le procédé selon l'invention repose sur une externalisation des réactions catalytiques dans le périplasme de bactéries. Les bactéries didermes à Gram négatif sont en premier lieu concernées, mais tout autre organisme possédant un périplasme peut aussi être utilisé. Selon une caractéristique préférée de l'invention, les n bactéries génétiquement modifiées introduites dans le mélange réactionnel sont des bactéries didermes, à Gram négatif, choisies chacune dans la famille des Enterobacteriaceae, des Alcaligenaceae, Vibrionaceae, Pseudomonadaceae, pour lesquelles la production de protéines recombinantes a déjà été décrite (Stock et al., 1977 ; Eichmann et al., 2019 ; Karyolaimos *et al.*, 2019). Elles peuvent notamment être des entérobactéries appartenant aux genres Salmonella, Yersinia ou Escherichia. Selon un mode de réalisation encore préféré, les bactéries sont de l'espèce Escherichia coli.

Ces bactéries sont aptes à exprimer dans leur espace périplasmique une des enzymes E1, E2, ..., En, chaque bactérie exprimant une enzyme différente des enzymes exprimées par les autres bactéries du consortium. Pour ce faire, une modification génétique a été pratiquée pour introduire dans les cellules une séquence nucléique codant l'enzyme concernée, associée à une séquence nucléique codant un peptide signal. Chaque bactérie est alors productrice d'un polypeptide dans lequel sont fusionnées deux séquences d'acides aminés : celle constituant l'enzyme et celle du peptide signal. Le polypeptide de fusion est ainsi adressé dans l'espace périplasmique, où le peptide signal est éliminé. Les enzymes se trouvent alors à l'état libre mais confinées dans le compartiment périplasmique, sans avoir la capacité de traverser la paroi externe. Il a été vérifié qu'elles se maintenaient dans une conformation active et qu'elles assuraient parfaitement leur fonction catalytique, chacune à partir des substrats disponibles dès le départ ou apparaissant au fur et à mesure de l'avancement des réactions. Différents peptides signaux peuvent être utilisés dans le cadre de la présente invention, tels que pelB, dsbA, EOX, lamB, MgIB, MmAp, ompC, ompT, sufl, SfmC, STII, tolB, torA, torT, glll, malE, ompA, phoA, ou d'autres encore choisis parmi ceux connus de l'homme de l'art, qui sait les utiliser pour permettre l'expression périplasmique (Karyolaimos *et al.*, 2019).

Ainsi selon l'invention, chacune des bactéries est génétiquement modifiée pour exprimer un polypeptide, comprenant respectivement une desdites enzymes E1, E2, ..., En, liée à un peptide signal adressant ledit polypeptide dans l'espace périplasmique de ladite bactérie.

Selon un autre mode de réalisation, les enzymes ou certaines d'entre elles peuvent être ancrées à la paroi périplasmique. Dans ce cas, selon l'invention, l'une ou plusieurs desdites n bactéries sont génétiquement modifiées pour exprimer un polypeptide comprenant respectivement une desdites enzymes E1, E2, ..., En, liée à un peptide d'ancrage membranaire incluant ledit peptide signal.

La transformation des bactéries est réalisée par insertion de la séquence nucléique adéquate à l'aide d'un vecteur convenable. Une technique consiste à faire pénétrer dans le cytoplasme bactérien un plasmide comportant une séquence nucléique apte à coder le polypeptide désiré. Une autre technique repose sur l'insertion d'une séquence nucléique dans le génome bactérien lui-même. Ces techniques et d'autres, sont connues dans le domaine des biotechnologies cellulaires, et seront mises en œuvre sans difficulté par une personne qualifiée dans le domaine concerné.

Une fois transformé, chaque variant est mis en culture pour la production de biomasse, puis utilisé immédiatement ou conservé. Il est commode de produire une série de bactéries aptes à exprimer différentes d'enzymes, de manière à disposer d'autant de modules pour construire des schémas réactionnels pouvant être mis en œuvre par le procédé selon l'invention. On pourra ainsi puiser dans cette banque cellulaire pour préparer à façon le mélange réactionnel désiré à l'étape a) du procédé selon l'invention.

Une fois que le mélange réactionnel est préparé dans le milieu convenable, on laisse les réactions se dérouler. Cette façon de procéder correspond à un mode de production discontinu (en batch). A noter qu'une production en mode continu ou semi-continu est tout à fait possible aussi comme il sera exposé plus loin. La chaine réactionnelle s'interrompt spontanément lorsque le substrat organique initial est épuisé. Le composé d'intérêt synthétisé se trouve pour l'essentiel excrété sous forme solubilisée dans le milieu aqueux. Selon le schéma réactionnel adopté, le composé sera constitué par l'un des produits P1, P2, ..., Pn obtenu dans l'une ou l'autre des réactions du schéma réactionnel. Après séparation de la biomasse, on pourra alors le récupérer aisément dans le surnageant, dont la composition est simple comparée à celle résultant des autres techniques connues jusqu'à présent.

Selon un mode de réalisation particulier de l'invention, le procédé peut utiliser deux modules cellulaires, dont le premier transforme le substrat organique initial, et le second utilise le produit de la première réaction pour donner un deuxième produit, que l'on peut séparer du milieu pour obtenir le composé désiré. Dans ce cas, le mélange réactionnel comprend une bactérie exprimant une première enzyme E1 apte à catalyser la première réaction R1 à partir dudit substrat organique pour fournir un premier produit P1, et une bactérie exprimant une deuxième enzyme E2 apte à catalyser une deuxième réaction R2 à partir dudit premier produit P1, pour former un deuxième produit P2, lequel est récupéré à l'étape c) en tant que composé d'intérêt.

Cette modalité peut être le début d'un schéma réactionnel plus étendu, auquel cas le deuxième produit peut être à son tour consommé en tant que substrat d'une réaction en aval. Dans un tel schéma réactionnel étendu, le mélange réactionnel comprend en outre au moins une bactérie exprimant une enzyme E3, ..., En, apte à catalyser une réaction R3, ..., Rn, à partir d'un produit Pn-1 obtenu par une réaction Rn-1, pour fournir un produit Pn, lequel est récupéré à l'étape c) en tant que composé d'intérêt ou est consommé en tant que substrat d'une réaction Rn+1.

Lorsqu'un cofacteur est requis pour effectuer la première réaction, on introduit celui-ci à l'étape a) dans le milieu réactionnel. Le premier module cellulaire est choisi pour transformer de substrat organique initial et ce cofacteur (dit premier cofacteur CoF1), et le second module va utiliser soit le produit P1 de la première réaction, pour donner un deuxième produit P2, soit le coproduit CoP1 de la première réaction pour donner un deuxième coproduit CoP2. Le produit P2 peut être séparé du milieu pour obtenir le composé désiré, ou bien impliqué en tant que substrat dans une réaction en aval d'un schéma réactionnel plus étendu. Selon une modalité particulière, le coproduit CoP2 peut également être impliqué en tant que substrat d'une autre réaction.

Ainsi, conformément à un mode de réalisation du procédé selon l'invention, le mélange réactionnel comprend un cofacteur CoF1 dudit substrat organique, une bactérie exprimant une enzyme E1 apte à catalyser une première réaction R1 à partir dudit substrat organique et du cofacteur CoF1 pour former un premier produit P1 et un premier coproduit CoP1 ; et une bactérie exprimant une deuxième enzyme E2 apte à catalyser une deuxième réaction R2 à partir du premier produit P1 pour former un deuxième produit P2, ou à partir du premier coproduit CoP1 pour former un deuxième coproduit CoP2, l'un des deux au moins étant consommé dans une troisième réaction, ou récupéré à l'étape c) en tant que composé d'intérêt. On comprend que l'expression "troisième réaction" désigne ici l'une quelconque des réactions pouvant avoir lieu en aval de la réaction visée.

Lorsque le schéma réactionnel est plus étendu, le mécanisme ci-dessus peut être généralisé. En effet, chacune des réactions Rn d'un schéma réactionnel peut mettre en œuvre un cofacteur CoFn réagissant avec le produit Pn-1 généré par la réaction Rn-1 en amont sous l'effet d'une enzyme En. Un produit Pn et un coproduit CoPn sont alors formés. Le coproduit CoPn peut ensuite être utilisé comme cofacteur CoFn+1 d'une réaction Rn+1 en aval. De son côté, le produit Pn peut être utilisé comme substrat d'une (autre) réaction Rn+1 en aval, à moins qu'il ne soit le composé d'intérêt que l'on cherche à obtenir. Cette modalité peut être généralisée à l'un ou plusieurs des modules impliqués.

C'est pourquoi, selon un mode de réalisation du procédé objet de l'invention, le mélange réactionnel comprend au moins un cofacteur CoFn, au moins une bactérie exprimant une enzyme En apte à catalyser une réaction Rn à partir du produit Pn-1 d'une réaction Rn-1 et dudit cofacteur CoFn pour former un produit Pn et un coproduit CoPn, l'un des deux au moins étant consommé dans une réaction Rn+1, ou récupéré à l'étape c) en tant que composé d'intérêt.

On l'a vu, une réaction Rn du schéma réactionnel peut utiliser en tant que substrat un cofacteur, qui est généralement un coproduit généré par une réaction en amont. Il convient dans ce cas que soit présent à ses côtés un autre composé, dit "substrat complémentaire", qui va donner un produit complémentaire à l'issu de la réaction Rn. Ce dernier peut ne pas être utile dans la suite du schéma réactionnel et s'accumuler dans le milieu. C'est pourquoi, selon une caractéristique du procédé objet de l'invention, le mélange réactionnel peut comprendre en outre au moins un substrat complémentaire SCn dudit au moins un cofacteur CoFn.

Dans le procédé selon l'invention, ledit au moins un cofacteur CoFn peut être introduit en totalité ou en partie dans le mélange réactionnel à l'étape a), ou durant l'étape b). Il est en effet parfois préférable d'ajouter un cofacteur CoFn n'intervenant pas dans les premières étapes du schéma réactionnel, seulement quand le produit Pn devant réagir avec ledit cofacteur CoFn est présent dans le milieu en quantité significative. La cinétique réactionnelle est ainsi prise en compte pour optimiser la production du composé d'intérêt.

Par ailleurs, lorsqu'un cofacteur est nécessaire à une première réaction, il est particulièrement avantageux qu'une seconde réaction (réaction en aval) permette sa régénération. En effet, les cofacteurs sont des composés onéreux qui impactent directement les coûts de production. Un schéma réactionnel a été imaginé pour répondre à cet objectif, dans lequel la seconde réaction consomme le coproduit issu de la première réaction en tant que cofacteur dans la deuxième réaction, pour reformer le cofacteur initial. Ce faisant, il convient d'apporter un substrat complémentaire de la deuxième réaction, qu'on choisira de sorte qu'il ait un prix de revient modéré. Au terme de la deuxième réaction, un second produit est formé qui peut présenter un intérêt ou pas, mais surtout le cofacteur de la première réaction est régénéré.

Ainsi dans un mode de réalisation particulièrement intéressant du procédé selon la présente invention, ledit au moins un cofacteur CoFn est formé au moins en partie dans le mélange réactionnel par une réaction Rn-1 catalysée par une enzyme En-1 apte à former un produit Pn-1 et un coproduit CoPn-1, ledit coproduit CoPn-1 étant identique au cofacteur CoFn.

Il est cependant préférable d'apporter une certaine quantité de ce cofacteur dans le milieu, afin d'initier le processus jusqu'à ce que la régénération du cofacteur soit suffisante pour assurer un apport continu dudit cofacteur. Or, il est apparu qu'une quantité minime suffit pour amorcer le cycle des réactions. La quantité du cofacteur qu'il est nécessaire d'introduire dans le mélange réactionnel pour un déroulement complet de la réaction concernée est alors significativement minorée par rapport à la quantité du substrat correspondant avec lequel il doit réagir. Elle peut être au moins 20 fois inférieure, mais de préférence au moins 100 fois inférieure, de manière encore préféré d'au moins 500 fois inférieure, et encore mieux d'au moins 1000 fois inférieure. A noter que certains cofacteurs synthétisés dans le cytoplasme cellulaire, tels que l'ATP par exemple, peuvent être présents dans l'espace périplasmique en quantité certes faible, mais suffisante.

Ainsi dans ce cadre, selon l'invention, la quantité du premier cofacteur CoF1 introduite dans le mélange réactionnel à l'étape a) correspond à une concentration molaire au moins 20 fois inférieure à la concentration molaire initiale dudit substrat organique.

Comme indiqué précédemment, le substrat organique initial peut être de nature diverse, incluant toute sorte de molécules carbonées. Le procédé a en particulier démontré son efficacité pour transformer des hydrates de carbone communs (sucres) tels que le glucose, le fructose, le glycérol, ou autres, en des composés d'intérêt tels que des sucres rares notamment des sucres phosphorylés, des isomères particuliers de sucres rares, du dihydroxyacétone et d'autres molécules recherchées pour donner accès à d'importantes voies de synthèse. D'autres composés organiques peuvent constituer le substrat de départ, parmi lesquels les aldéhydes, les alcools, les acides organiques, les carbamates, les hydrocarbures, les acides aminés. A titre d'exemple peuvent être cités le glyoxal fournissant du glycolaldéhyde, ou encore la L-phénylalanine point de départ de production du 2-phényléthanol. Le substrat organique peut aussi être par extension, un composé comprenant un unique atome de carbone, tel que le dioxyde carbone.

Ainsi, selon une caractéristique du procédé objet de la présente invention, le substrat organique de la première réaction est choisi parmi les hydrates de carbone, les aldéhydes, les alcools, les acides organiques, les carbamates, les hydrocarbures, les acides aminés, le dioxyde de carbone.

De nombreuses enzymes peuvent être produites par les différentes bactéries transformées mises en œuvre dans le procédé inventif. Une personne du métier sait identifier celles convenant à l'élaboration d'un schéma réactionnel menant d'un substrat organique à un composé d'intérêt donnés. Il peut les sélectionner parmi celles qui sont répertoriées et décrites ou qui seront identifiées à l'avenir.

Ainsi, conformément à une caractéristique préférée du procédé selon l'invention, les enzymes E1, E2, ..., En, sont chacune choisies parmi les kinases, les déshydrogénases, les phosphatases, les réductases, les isomérases, les transférases. Les kinases peuvent être notamment une glycérol kinase, une glucokinase, une fructokinase, une xylokinase, une acétate kinase, ou une phosphofructokinase. Les déshydrogénases sont par exemple la glycérol déshydrogénase, ou la phosphite déshydrogénase. Des réductases telles que la NADH réductase, la glyoxal réductase peuvent être utilisées. Les isomérases peuvent être choisies parmi une xylose isomérase, une xylulose isomérase, une ribulose-phosphate-3-épimérase, une ribose-5-phosphate isomérase, tandis qu'une transcétolase, ou une transaldolase peuvent être choisies comme transférase. Diverses phosphatases sont également connues.

Par ailleurs, de multiples cofacteurs peuvent être impliqués dans l'une ou l'autre des réactions du schéma réactionnel. A noter que les cofacteurs formant des coproduits qui peuvent à leur tour jouer le rôle de cofacteur dans une réaction en amont ou en aval, les molécules concernées appartiennent aux deux catégories. Ainsi, de préférence, dans le procédé selon l'invention, l'une au moins des enzymes E1, E2, ..., En, catalyse une réaction fournissant un des coproduits CoP1, CoP2, ..., CoPn, choisi parmi l'ATP, l'ADP, l'AMP, l'UTP, l'UDP, l'UMP, le NAD+, le NADH, le NADP+, le NADPH, le FAD, le FADH2, le coenzyme A, ou catalyse une réaction utilisant l'un d'eux comme cofacteur.

On a vu qu'à l'issue de l'étape c) du procédé inventif, la biomasse est séparée du surnageant duquel le composé d'intérêt est extrait. Or, les travaux réalisés ont mis en évidence un avantage inattendu du procédé inventif. Il est en effet apparu que cette biomasse, contenant les cellules des n bactéries apportées à l'étape a), peut être réutilisée telle quelle dans un nouveau cycle de réactions, avec un rendement de même niveau et même parfois supérieur à celui observé lors du premier cycle réactionnel. On réalise de ce fait un gain important en termes de temps, de nombre de manipulations à effectuer, de consommation d'ingrédients et d'eau et finalement, en terme économique. Cet avantage découle du principe de l'invention selon lequel la phase de culture cellulaire est dissociée de la phase de synthèse biochimique : elles se déroulent dans des réacteurs distincts, avec des milieux différents.

Le surnageant peut être séparé de la biomasse par écoulement gravitaire, et dans ce cas, il est possible de réutiliser directement la biomasse restée dans le réacteur, en y ajoutant seulement du substrat frais et optionnellement les autres ingrédients nécessaires.

Ainsi, de manière particulièrement avantageuse, après l'étape c) du procédé objet de la présente invention, la biomasse séparée du surnageant est réutilisée pour préparer un nouveau mélange réactionnel selon l'étape a), avec ou sans étape de conservation intermédiaire.

Le procédé pourra être mis en œuvre dans le cadre de cultures discontinues par lots (dites en batch) comme décrit ci-dessus, ou à écoulement discontinu (de type fed batch). Il peut aussi être conduit en régime semi-continu ou avec alimentation continue, selon les règles de l'art connues. Dans ce cas, les étapes a) et b) ne se déroulent pas de manière séquentielle, mais de manière plus ou moins simultané.

C'est pourquoi, dans une variante de réalisation du procédé selon l'invention, les étapes a) et b) sont réalisées en ajoutant dans le mélange réactionnel contenant lesdites n bactéries, en permanence ou à intervalle de temps,
- ledit substrat organique, et
- optionnellement, un cofacteur dudit substrat organique CoF1, un cofacteur CoFn desdits produits obtenus par une réaction Rn-1; et/ou un substrat complémentaire SC d'un coproduit obtenu par une réaction Rn-1,
et en laissant réagir le mélange réactionnel ainsi obtenu en mode continu ou semi-continu.

Il ressort de ce qui précède que l'approche adoptée par le procédé de production de composés d'intérêt tel qu'il vient d'être décrit, présente de nombreux avantages répondant aux difficultés rencontrées avec les techniques conventionnelles et revêt une portée très générale. L'externalisation des réactions permet en effet d'utiliser la machinerie biochimique de la cellule pour fournir les effecteurs enzymatiques de réactions se déroulant dans l'espace délimité du périplasme, ceci tout en laissant circuler les réactifs et produits mis en jeu dans les réactions. La conception par modules combinables permet d'élaborer des schémas réactionnels originaux complets et de les mettre en œuvre rapidement. La synthèse d'un produit d'intérêt donné est ainsi facilitée, mais aussi sa purification finale, puisque le milieu aqueux de bioproduction est très simple.

Il faut ajouter que le procédé reposant sur la production de biomasse réutilisable, il est bien moins couteux que les procédés enzymatiques connus (quand ils existent). Il est dès lors possible de produire industriellement des molécules de toutes sortes, y compris complexes ou difficiles d'accès.

On comprend ainsi que le procédé objet de l'invention représente une alternative inédite aux méthodes de production actuelles, et offre un puissant outil permettant d'accélérer le développement de nouveaux produits.

Selon un deuxième aspect, la présente invention concerne un mélange réactionnel pour la production d'un composé d'intérêt à partir d'un substrat organique. Un tel mélange réactionnel est essentiellement identique à celui qui est préparé à l'étape a) du procédé de production décrit plus haut. Le mélange réactionnel est tel que défini dans l'objet de la revendication 13.

Ainsi, la présente invention a pour objet un mélange réactionnel pour la production d'un composé d'intérêt à partir d'un substrat organique, contenant, dans un milieu convenable qui est un milieu aqueux comme décrit précédemment :
- n bactéries, n étant un nombre entier au moins égal à 2, chaque bactérie étant génétiquement modifiée pour exprimer dans son espace périplasmique une enzyme E1, E2, ..., En, chacune desdites n bactéries exprimant une enzyme différente de celle exprimée dans les autres bactéries,
   - une enzyme E1 étant apte à catalyser une réaction R1 à partir dudit substrat organique pour fournir un produit P1, et optionnellement un coproduit CoP1,
   - chacune desdites enzyme E2, ..., En, étant apte à catalyser une réaction R2, ..., Rn, à partir d'un produit ou d'un coproduit obtenu par une réaction Rn-1, pour fournir respectivement un produit P2, ..., Pn, et optionnellement un coproduit CoP2, ..., CoPn,
- ledit substrat organique, et
- optionnellement, un cofacteurCoF1 dudit substrat organique, un cofacteur CoFn desdits produits obtenus par une réaction Rn-1,; et/ou un substrat complémentaire SC d'un coproduit obtenu par une réaction Rn-1.

Dans le mélange réactionnel de l'invention tel qu'objet de la revendication 13 :
lesdites n bactéries génétiquement modifiées sont des bactéries didermes à Gram négatif, choisies chacune dans la famille des Enterobacteriaceae, des Alcaligenaceae, des Vibrionaceae, ou des Pseudomonadaceae, et
chacune desdites n bactéries est génétiquement modifiée pour exprimer un seul polypeptide, comprenant respectivement une desdites enzymes E1, E2, ..., En, liée à un peptide signal adressant ledit polypeptide dans l'espace périplasmique de ladite bactérie ou un peptide d'ancrage membranaire incluant ledit peptide signal.

Selon l'invention revendiquée, chaque bactérie est génétiquement modifiée pour exprimer dans son espace périplasmique une seule enzyme E1, E2, ..., En, chacune desdites n bactéries exprimant une enzyme différente des autres bactéries.

Le mélange réactionnel est donc une composition mixte comprenant les cellules en suspension d'au moins deux bactéries, et différents réactifs solubles, dont le substrat organique à transformer pour obtenir le composé d'intérêt. On pourra se référer à l'exposé précédent pour définir les diverses modalités de réalisation de ce mélange. Selon un mode particulier, le mélange réactionnel comprend au moins 3 bactéries, notamment au moins 4 bactéries, voire au moins 5 bactéries, et en particulier au moins 6 bactéries, chaque bactérie étant génétiquement modifiée pour exprimer une enzyme différente des enzymes exprimées par les autres bactéries.

Enfin, selon un troisième aspect de la présente invention, est revendiquée l'utilisation d'un mélange réactionnel tel que décrit ci-dessus, pour produire un composé d'intérêt choisi parmi les hydrates de carbone, les aldéhydes, les alcools, les acides organiques, les carbamates, les hydrocarbures, les acides aminés. A titre d'exemple, un mélange réactionnel comprenant un hydrate de carbone peut être utilisé pour produire un sucre phosphorylé ou du DHA (dihydroxyacétone), ou encore un mélange réactionnel comprenant un acide aminé peut être utilisé pour produire un composé organique azoté.

La présente invention sera mieux comprise et des détails en relevant apparaîtront à la lumière de la description qui va être faite de différents modes de réalisation, en relation avec les figures annexées.
Fig.1 : Production de glycérol-3-phosphate au cours du temps à partir de glycérol par deux modules cellulaires (BL21-Glpk et BL21-AckA).
Fig.2a : Effet de la concentration en modules cellulaires (BL21-Glpk et BL21-AckA) sur la production de glycérol-3-phosphate.
Fig.2b: Effet du volume réactionnel - production de glycérol-3-phosphate dans des volumes de 2 ml, 20 ml, 200 ml et 2000 ml.
Fig.3a : Production de D-glucose-6-phosphate à partir de D-glucose par deux modules cellulaires BL21-Glk et BL21-AckA.
Fig.3b : Production de D-glucose-6-phosphate à partir de D-glucose par deux modules cellulaires BL21-nlpA-Glk et BL21-nlpA-ackA.
Fig.4 : Production de fructose-1-phosphate à partir de D-fructose par deux modules cellulaires BL21-KhkC et BL21-AckA.
Fig.5 : Production de glucose-6-phosphate avec réutilisation des modules cellulaires BL21-GIK et BL21-AckA.
Fig.6 : Production de dihydroxyacétone à partir de glycérol par les modules BL21-GldA et BL21-Nox.
Fig.7 : Production de glycolaldéhyde à partir de glyoxal par le module BL21-YvgN, avec ou sans module de régénération BL21-PtxD.
Fig.8 : Consommation de D-xylose par la réaction d'isomérisation seule (BL21-XylA) et par la réaction totale (BL21-XylA, BL21-XyIB, BL21-AckA, BL21-AphA).
Fig.9 : Production de D-xylulose à partir de D-xylose par la réaction d'isomérisation seule (BL21-XyIA) et par la réaction totale (BL21-XylA, BL21-XylB, BL21-AckA, BL21-AphA).
Fig.10 : Migration sur gel SDS-PAGE des fractions périplasmiques des modules XyIA, XyIB, AcKA, Rpe, RpiA, TktA, Tal, PfkA et du contrôle BL21 WT.
Fig.11 : Production de D-fructose-1,6-bisphosphate à partir de D-xylose à l'aide de huit modules cellulaires.
Fig.12 : Production de l'intermédiaire D-sedoheptulose-7-phosphate au cours de la production de D-fructose-1,6-bisphosphate avec huit modules.
Fig.13. Suivi de la concentration de 2-phénylethanol par HPLC dans un consortia synthétique composé des souches BL21-ARO8, BL21-ARO10, BL21-ADH5 et BL21-PtxD et dans le contrôle BL21-WT en présence de 10 mM de L-phénylalanine.
Fig. 14. Gel SDS-PAGE des fractions périplasmiques des biocatalyseurs BL21-AckA, BL21-GIpK, BL21-AckA-GlpK et du contrôle négatif BL21- W.
   M : Marqueur Precision Plus Protein ^{™} All blue, BIO-RAD ^{®} ; 1: BL21-AckA (2 DO₆₀₀ₙₙ/ml) ; 2: BL21-GlpK (2 DO₆₀₀ₙₘ/ml) ; 3 : BL21-GlpK-AckA (2 DO₆₀₀ₙₘ/ml), 4 : BL21-GIpK (1DO₆₀₀ₙₘ/ml), BL21-GlpK ( 1DO₆₀₀ₙₘ/ml), 5 :BL21-WT (2DO₆₀₀ₙₘ/ml) ; cadre tirets noirs : protéine GlpK de 56 kDa ; cadre en tirets blancs : protéine AckA de 43 kDa.

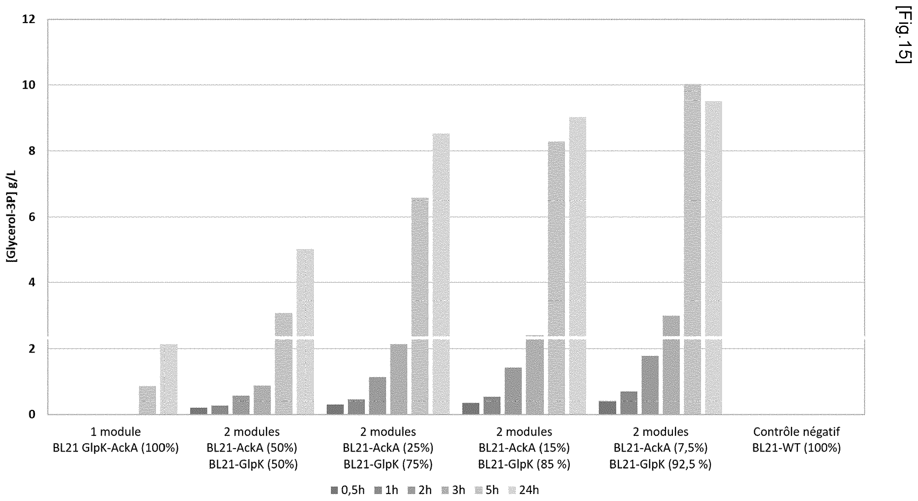

Fig. 15. Production de glycérol-3-phosphate à partir de glycérol par la souche BL21-GlpK-AckA, le consortia synthétique composé des 2 modules BL21-AckA et BL21-GlpK apportés en proportions variables et le contrôle BL21-WT.

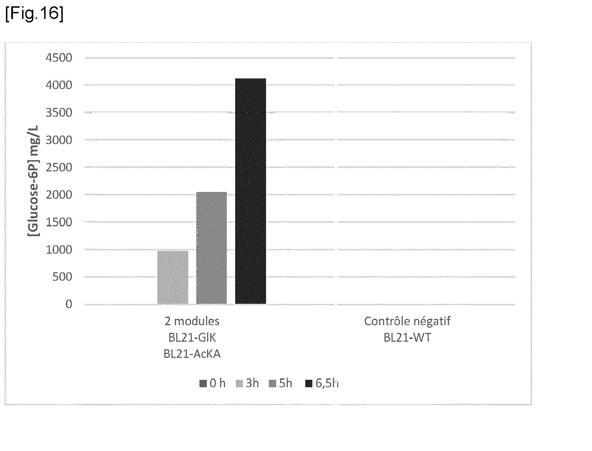

Fig.16. Production de glucose-6-phosphate dans un consortia synthétique composé de 2 modules BL21-Glk, BL21-AckA et dans le contrôle BL21-WT en présence de glucose et sans ajout d'ATP.

### MATERIEL ET METHODES

### 1- Plasmides

- Le plasmide pET22b (ori pBR322, ampicilline, promoteur T7, pelB) et le plasmide pET26b (ori pBR322, kanamycine, promoteur T7, pelB) sont des plasmides commerciaux portant la séquence signal pelB en N-terminal et une séquence His-tag en C-terminal. Ils ont été utilisés pour l'expression d'un gène d'intérêt codant pour une enzyme qui sera transportée grâce au peptide fusion PelB dans le périplasme de la cellule.
- Le plasmide pACT3 (ori p15A, chloramphénicol, promoteur Ptac) a servi à l'ancrage des protéines dans le périplasme. Le plasmide a été modifié pour avoir la séquence leader de la lipoprotéine NIpA en N-terminal.
- Le plasmide pETDuet-1 (ori pBR322, ampicilline, promoteur T7) est conçu pour la co-expression de deux gènes d'intérêt. Le vecteur porte deux sites de clonage, chacun étant précédé d'un promoteur T7, un opérateur lac et une séquence Shine-Dalgarno. Le plasmide pETDuet-1 a ici été modifié en intégrant après les deux sites de fixation au ribosome la séquence signal PelB. Les cassettes d'expression (T7-RBS-pelB) sont identiques à celle de pET22b. Les gènes intégrés dans ce plasmide modifié permettent la co-expression de deux enzymes libres dans le périplasme sous le contrôle de deux promoteurs T7 inductibles à l'IPTG.

Les gènes sont amplifiés depuis l'ADN génomique d'Escherichia coli MG1655 par une technique connue en soi, par exemple par PCR (Polymerase Chain Reaction) à l'aide des amorces adéquates, puis insérés dans les plasmides pET22b, pET26b, pACT3 ou pETDuet-1 préalablement linéarisés avec le kit NEBuilder^{®} par recombinaison homologue. Si précisé, des gènes synthétiques ont directement été commandés chez Genscript^{®} et directement clonés par leur soin dans le plasmide pET22b ou pET26b.

Les amorces utilisées pour l'amplification génique sont de type généralement connu. L'homme de l'art sait les choisir et les utiliser dans le cadre des techniques de PCR. Selon un mode particulier, les amorces pour l'amplification de chacun des gènes sont préparées en utilisant le kit et les instructions du fabriquant NEB « NEBuilder HiFi DNA Assembly Master Mix & NEBuilder HiFi DNA Assembly Cloning Kit E2621 E5520 » (https://www.neb.com/- /media/nebus/files/manuals/manuale2621_e5520.pdf?rev=67d09a762b2c47ed8df3a 0bd1cb59a1 b&hash=02CF464149634283F5633CA1DE4D2FDF).En particulier et selon les recommandations du fabriquant, les amorces PCR à utiliser dans l'assemblage d'ADN HiFi doivent avoir deux composants de séquence : i) une séquence de chevauchement, nécessaire à l'assemblage de fragments adjacents ; ii) une séquence spécifique au gène, requise pour l'amorçage de la matrice pendant la PCR. Pour obtenir un assemblage efficace des fragments de PCR dans un vecteur, nous il est suggéré d'utiliser un chevauchement de 15 à 30 nucléotides avec une Tm égale ou supérieure à 48°C (en supposant que la paire A-T = 2°C et la paire G-C = 4°C). Sont citées à titre d'exemple se rapportant aux plasmides susmentionnés, les amorces suivantes : GLPK_pET22b ; Khk-C_pET22b ; ackA_pET26b ; gldA_pET22b ;; XylA_pET22b ; XyIB_pET22b ; GLK_pET22b ; GLK_pET22b ; pET22B_rpe ; pET22B_rpiA; pET22b_tktA ; pET22b_tal ; pET22b_pfkA ; nlpA_glk ; pACT3_glk ; nlpA_ackA ; pACT3_ackA ; pETDuet_glpK_ack ;pET22b_ARO8 ; pET22b_ARO10 ; pET22b_ADH5.

### 2 - Construction des souches

La souche BL21(DE3) ou BL21(DE3) gold ont été choisies pour la transformation des plasmides pET22b ou pET26b (en fonction de la cassette de résistance) afin de pouvoir utiliser la T7 polymérase. Les transformations ont été réalisées en suivant le protocole TSS décrit par Chung and Miller (Chung et coll., 1993). Les deux dernières souches ont été construites pour que l'enzyme soit ancrée dans le périplasme, toutes les autres pour qu'elle soit libre. Les souches utilisées sont données dans le Tableau 1.

**[Tableau 1] Souches d'Escherichia coli utilisées**

| Gène surexprimé (Organisme) | Gene ID (NCBI) | Genbank | Nom du module | Plasmide | Résistance | Souche : Génotype |
|---|---|---|---|---|---|---|
| xylA (E.coli) | 948141 | X0772.1 | BL21-XylA | pET22b - xylA | Amp | BL21 (DE3) : |
| xylB (E.coli) | 948133 | X04691.1 | BL21-XylB | pET22b - xylB | | |
| ackA (E.coli) | 946775 | M22956.1 | BL21-AckA | pET26b-ackA | Kan | E. coli str. B F-ompT gal dcm Ion hsdSB(rB-mB-) A(DE3 [lacl lacUV5-T7p07 ind1 sam7 nin5]) |
| rpe (E.coli) | 947896 | Z19601 | BL21-Rpe | pET22b **-** rpe | Amp | |
| rpiA (E.coli) | 947407 | X73026 | BL21-RpiA | pET22b - rpiA | | |
| tktA (E.coli) | 947420 | X68025 | BL21-TktA | pET22b - tktA | | |
| talA (E.coli) | 947006 | D13159 | BL21-TalA | pET22b - talA | | |
| pfkA (E.coli) | 948412 | X02519 | BL21-PfkA | pET22b - pfkA | | |
| Nox (Lactobacillus sanfransiscensi s) | | CP002461.1 | BL21-Nox | pET22b - nox | | |
| gldA (E.coli) | 948440 | U00006 | BL21-GIdA | pET22b - gldA | | |
| yvgN (B.subtilis) | 936001 | AJ223976 | BL21-YvgN | pET22b - yvgn | | |
| | | | | | | |
| glk (E.coli) | 946858 | U22490 | BL21-Glk | pET22b - glk | | |
| glpK (E.coli) | 948423 | M18393 | BL21-GIpK | pET22b - glpk | | |
| khkC (H.sapiens) | 3795 | X78678 | BL21-KhkC | pET22b - khkc | | |
| ptxD (Pseudomonas stutzeri) | 69882623 | AF061070 | BL21-PtxD | pET22b - ptxD | | |
| aphA (E.coli) | 948562 | U51210 | BL21-AphA | pET22b-apha | | |
| / | | | BL21-WT | pET22b-MSC | | |
| glpK (E.coli) ackA (E.coli) | 948423 - 946775 | M18393-M22956.1 | BL21-GlpK-AckA | pETDuet1-peIB-glpK-peIB-ackA | | |
| ARO8 (S.cerevisiae) | 852672 | Y13624 | BL21-ARO8 | pET22b-ARO8 | | |
| ARO10 (S.cerevisiae) | 851987 | U28373 | BL21-ARO10 | pET22b-ARO10 | | |
| ADH5 (S.cerevisiae) | 852442 | Z36014 | BL21-ADH5 | pET22b-ADH5 | | |
| glK (E.coli) | 946858 | U22490 | BL21-nlpA-Glk | pACT3-nlpA-glk | Chm | |
| ackA (E.coli) | 946775 | M22956.1 | BL21-nlpA-AckA | pACT3-nlpA-ackA | | |

### 3 - Milieux et conditions de culture

### Production de cellules entières induites

Les souches sont reprises à partir d'un stock glycérol conservé à -80°C, dans 5 ml de milieu TB tamponné à pH 7 avec l'antibiotique adapté, et placées à 37°C une nuit. La composition du milieu TB est donnée au Tableau 2.

**[Tableau 2] Composition du milieu TB tamponné à pH 7**

| **Composé** | **Quantité** | **Unité** |
|---|---|---|
| **Extrait de levure** | 24 | g/l |
| **Tryptone** | 20 | g/l |
| **Glycérol** | 5 | g/l |
| **Kh2PO4** | 0,017 | M |
| **Na2HPO4** | 0,072 | M |

Le lendemain, la préculture est diluée au 1/100ème dans 50 ml de milieu TB frais, la culture est agitée à 200 rpm à 37°C. Lorsque la densité optique à 600 nm (DO600nm) atteint une valeur comprise entre 0,4 et 0,6, les cellules sont induites avec 0,1 mM d'IPTG. Elles sont alors placées à 20°C sous agitation à 200 rpm, pendant environ 18 heures.

### Production externalisée de molécules cibles

Les cellules entières induites exprimant une ou plusieurs enzymes dans leur périplasme (ou modules) sont centrifugées à 4500 rpm pendant 10 minutes et resuspendues dans de l'eau stérile afin d'atteindre une quantité de cellules définie exprimée en uDO. Les modules portant les enzymes, choisis selon les besoins d'un schéma réactionnel prédéfini, sont introduits ensemble dans le mélange réactionnel. Celui-ci comprend un tampon afin de stabiliser le pH, si nécessaire des activateurs et des cofacteurs, le substrat et une quantité connue de chaque module cellulaire. La composition est volontairement la plus simple possible afin d'assurer une activité enzymatique optimale et un coût de production minimisé. Les substrats et les cofacteurs ont été achetés chez Sigma^{®} ou Carbosynth^{®}, hormis le phosphate d'acétyle utilisé pour la régénération de l'ATP qui a été synthétisé selon la méthode décrite dans (Crans et coll., 1983), à partir d'acide phosphorique (85%, 2 mol) et d'éthylacétate (2 mol), deux composés à faible coût.

### 4 - Quantification et analyse des produits extracellulaires

Le suivi des concentrations des substrats et des produits est réalisé par chromatographie ionique haute pression (HPIC - High Pressure Ion Chromatography) ou par chromatographie liquide haute performance (HPLC - High Perfomance Liquid Chromtography). Différentes méthodes de détection ont été utilisées en fonction des molécules cibles.

### Analyses HPIC

Les prélèvements sont centrifugés à 14800 rpm pendant 2 minutes, puis filtrés à 0,2 µm. Les échantillons à analyser sont dilués au 100ème dans de l'eau milli-Q dans des fioles équipées d'un bouchon pré-percé et placés à 15°C dans un passeur automatique d'échantillons.

### • Détecteur ampérométrique (HPIC - PAD)

L'analyse de certains substrats et produits se fait par chromatographie ionique (Dionex^{™} ICS-6000 ion chromatography system) couplée à la détection ampérométrique pulsée ou PAD (Dionex^{™} ICS-6000 Electrochemical Detector). Les molécules sont séparées sur une colonne Dionex^{™} Carbopac^{™} PA1 (2x250mm) précédée d'une pré-colonne de même type (2x50mm) à 25°C.

Deux méthodes d'élution ont été utilisées. La méthode utilisée pour le dosage des sucres (xylose, glucose, fructose), les composés organophosphorés (glucose-6P, fructose-1P, fructose-1,6bP, glycérol-3P), le méthylglyoxal et les aldéhydes tel que le glycolaldéhyde est la suivante : la concentration en NaOH est maintenue à 100 mM de 0 à 30 min tandis que la concentration d'acétate de sodium (NaOAc) varie graduellement. De 0 à 2 min, 0 mM de NaOAc ; de 2 min à 15 min augmentation de 0 à 500 mM de NaOAc ; de 15 min à 23 min concentration maintenue à 500 mM NaOAc ; de 23 min à 23,1 min diminution de 500 mM à 0 mM de NaOAc ; de 23,1 min à 30 min maintien à 0 mM NaOAc.

### • Détecteur conductimétrique (HPIC - CD)

L'analyse des anions (composés organophosphorés, phosphate, chlorure, sulfate, acides organiques...) se fait par chromatographie ionique (Dionex^{™} ICS-6000 ion chromatography system) équipée d'un conductimètre (Dionex^{™} ICS-6000 CD Conductivity Detector). Les molécules sont séparées sur une colonne Dionex^{™} IonPac^{™} AS11-HC (2x250mm) précédée d'une pré-colonne de même type (2x50mm) à 25°C.

Les gradients de concentration sont réalisés avec le générateur d'éluant EGC 500 KOH à un débit de 0,35 ml/min. Le gradient suivant a été utilisé pour séparer au mieux les composés intermédiaires du schéma réactionnel présenté dans l'exemple 7. Avant chaque injection un conditionnement de 7 min à 2 mM de NaOH est réalisé. La première étape du gradient est une élution isocratique à une concentration de NaOH de 10 mM pendant 3 min suivi d'une augmentation de la concentration en NaOH en plusieurs étapes : 1) augmentation de 10 mM à 50 mM en 9 min ; 2) augmentation de 50 mM à 100 mM en 7 min ; 3) maintien à 50 Mm pendant 3 min ; 4) diminution de la concentration en NaOH à 10 mM ; 5) maintien à 10 mM pendant 4 min. En sortie de colonne, un suppresseur ADRS_2mm (87mA) est utilisé afin d'améliorer la détection. Pour les autres exemples, le gradient de concentration en NaOH présenté ci-après a été utilisé pour le dosage des métabolites anioniques. La première étape est une élution isocratique à une concentration de NaOH de 2 mM pendant 4 min suivie d'une augmentation en plusieurs étapes de la concentration en NaOH : 1) augmentation de 2 mM à 100 mM en 11 min ; 2) maintien à 100 mM pendant 10 min ; 3) diminution à 2 mM et maintien pendant 1 min.

### Analyses UHPLC-UV/RI

L'analyse des sucres et des acides organiques se fait par UHPLC (Dionex Ultimate^{™} 3000) équipée d'un réfractomètre (Shodex^{™} RI-101) et d'un détecteur UV (Dionex UltiMate^{™} 3000 Diode Array Detectors 3000(RS)). Les molécules sont séparées sur une colonne Phenomenex^{™} ROA-Organic Acid H+ (8%) (300x7,8mm) avec pré-colonne de même type (50x7,8mm). L'élution se fait en isocratique (H2SO4 5mM) à un débit de 0,5 ml/min pendant 35 minutes. Les échantillons à analyser sont filtrés à 0,2 µm avant injection.

### Analyses HPLC - spectrométrie de masse

La présence du fructose-1-phosphate est détectée par UHPLC (Dionex UltimateTM 3000) équipée d'un spectromètre de masse (Thermo Scientific^{™}). Les molécules sont séparées sur une colonne ACQUITY UPLC BEH Amide Column (130Å, 1.7 µm, 2.1x30mm) précédée d'une précolonne (ACQUITY BEH Shield RP18 1.7µM VANGUARD). La phase mobile A est composée d'eau ultra pure et d'acide méthylphosphonique (1,8 mM), et la phase mobile organique B d'acétonitrile et d'acide formique (1,3 mM). Le gradient de concentration utilisé est le suivant, à un débit de 0,35 ml/min : 1 min en isocratique à 90 % de la phase mobile organique, suivi de diminutions de la fraction de la phase mobile organique en plusieurs étapes : 82 % à 3 min ; 78 % à 6,5 min ; 50 % à 8 min. Cette composition est maintenue constante pendant 10 min (50 % de phase organique) suivie d'un temps d'équilibrage de 7 min.

Le glycérol-3-phosphate C13 (G3P-C13) est utilisé comme étalon interne. Un gradient d'élution est réalisé. Le mélange injecté est préparé dans des inserts contenant 100 µl de l'échantillon filtré non dilué, 100 µl d'éluant (acétonitrile + acide formique 1,3mM) et 10 µl d'étalon interne (G3P-C13). La dilution finale de l'échantillon est de 2,1. Ce facteur de dilution est pris en compte dans le traitement des résultats avec le logiciel ChroméléonTM.

### 5 - Extraction des protéines par choc osmotique

Les cellules sont cultivées dans 2 ml de milieu LB une nuit durant, à 37°C et sous agitation à 200 rpm. La suspension cellulaire est transférée dans un tube Eppendorf et centrifugée (2 ml ; 5 min ; 14000 g ; 4°C). Le culot est resuspendu dans 500 µl du tampon N°1 et incubé 20 min sur glace. La place des tubes est inversée régulièrement pour éviter la sédimentation. Le mélange cellulaire est centrifugé (15 min ; 14000 g ; 4°C). Le surnageant est jeté et le culot cellulaire est resuspendu dans 125 µl de tampon N°2. Après une nouvelle incubation de 10 à 20 minutes sur glace, avec inversion régulière, les cellules sont centrifugées (15 min ; 14000 g ; 4°C). Le surnageant contenant la fraction périplasmique est récupéré, puis dénaturé pendant 10 min à 95°C et déposée sur gel SDS-PAGE (45 min, 180 V, 400 mA) pour vérifier la présence de l'enzyme et son poids moléculaire. Le marqueur de taille utilisé est le Precision Plus Protein^{™} All Blue Standards #1610373EDU. La composition des tampons est donnée dans le Tableau 3.

**[Tableau 3] : Composition des tampons N°1 et N°2**

| Tampon N°1 | Tampon N°2 |
|---|---|
| 0,2 M Tris - HCl (pH 8,0) | 0,01 M Tris HCl (pH 8,0) |
| 200 g/l sucrose | 0,005 g/l MgSO4 |
| 0,1 M EDTA | 0,2% SDS |
| | 1% Triton X-100 |
| | |

### EXEMPLE 1 : Production de composés phosphorylés

La phosphorylation est une réaction permettant aux cellules de séquestrer des métabolites essentiels dans le cytoplasme et d'éviter leur perte par diffusion dans le milieu extérieur. En effet, les composés phosphorylés chargés négativement ne peuvent pas diffuser à travers la bicouche phospholipidique. Ainsi, le catabolisme des sucres comprend toujours une étape de phosphorylation. Par exemple, les sucres tels que le D-glucose, D-fructose, D-thréalose, L-rhamnulose, le D-xylose et le L-arabinose sont respectivement piégés dans le cytoplasme sous forme de D-glucose-6-phosphate, D-fructose-1-phosphate, D-threalose-6-phosphate, L-rhamnulose-6-phosphate, D-sorbitol-6-phosphate, D-xylulose-5-phosphate et D-ribulose-5-phosphate. Il en est de même pour les autres sources de carbone assimilables par les microorganismes tels que les polyols : mannitol, sorbitol et glycérol respectivement phosphorylés en mannitol-1-phosphate, sorbitol-6-phosphate et glycérol-3-phosphate. L'externalisation de la réaction de phosphorylation permet de résoudre ce problème d'export et de réaliser une production microbienne de ces composés, conformément à la présente invention.

Pour ce faire, une souche d'Escherichia coli a été transformée pour exprimer dans son périplasme une kinase catalysant la phosphorylation d'un sucre (premier module). Cette réaction consomme un cofacteur, en l'occurrence de l'ATP, et forme de l'ADP en tant que coproduit. Un second module a été construit en transformant une souche d'Escherichia coli pour qu'elle exprime dans son périplasme l'acétate kinase (AckA). Cette enzyme catalyse une réaction utilisant du phosphate d'acétyle et de l'ADP pour former de l'acétate et de l'ATP.

Le schéma réactionnel est le suivant :

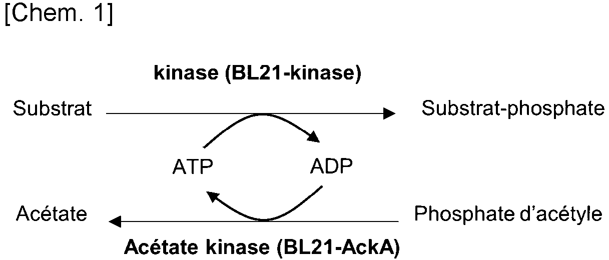

Les exemples ci-après illustrent la production de composés phosphorylés d'intérêt à partir de trois substrats organiques (glycérol, glucose, fructose), avec intervention des cofacteurs ATP et ADP, répondant à ce schéma réactionnel. Du fait qu'intervient le module BL21- AckA réalisant la régénération de l'ATP, la quantité d'ATP introduite dans le mélange réactionnel initial est nettement inférieure à celle du substrat (en l'occurrence 32 fois moindre pour l'un des exemples et 60 fois moindre pour les deux autres).

L'utilisation du module BL21- AckA permet ainsi de réaliser une économie substantielle car le coût de l'ATP est de l'ordre de 300 €/kg. Cependant, une autre raison importante motive la régénération du cofacteur ATP. En effet, certaines kinases sont inhibées par l'ADP. La glycérol kinase, par exemple, est inhibée par de faibles quantités d'ADP (Kic = 500 µM). Or, dans la première réaction, une molécule d'ADP est générée par molécule de produit formée. En cas d'inhibition par l'ADP, la production du composé d'intérêt est dès lors impossible sauf à éliminer en permanence l'ADP formé, ce qui est fait ici grâce à la réaction de régénérant l'ATP. L'emploi d'un module de régénération permet ainsi la production de molécules phosphorylées.

### EXEMPLE 1.1 : Production de glycérol-3-phosphate à partir de glycérol

Le glycérol-3-phosphate est le point d'entrée de la voie des phospholipides. Cette molécule d'intérêt a été produite à hauteur de 325 mg/l par voie microbienne en bioréacteur alimenté (ou fed-batch) mais son export s'est révélé être un problème majeur (Popp et al. 2008). En outre, un phénomène de déphosphorylation du glycérol se produit à l'intérieur de la cellule.

Deux modules sont utilisés : le premier module exprime la glycérol kinase d'E. coli qui produit du glycérol-3-phosphate à partir de glycérol et d'ATP, le second module a pour fonction de régénérer de l'ATP via l'acétate kinase d'E.coli AckA. Ces deux modules BL21-Glpk et BL21-AckA sont obtenus à partir d'un châssis BL21(DE3).

Les deux modules sont cultivés et induits à l'IPTG en milieu TB comme décrit (Matériel et méthodes, point 3) avant d'être récupérés pour effectuer la production de glycérol-3-phosphate dans un milieu simple. Le mélange réactionnel comprend :
Module 1 : 2 uDO/ml de cellules BL21-Glk
Module 2 : 2 uDO/ml de cellules BL21- AckA
Substrat : glycérol (160 mM)
Cofacteur : ATP (5 mM)
Substrat complémentaire : phosphate d'acétyle (220 mM)
Solution tamponnée : HEPES (50 mM) et MgSO4 (15 mM)

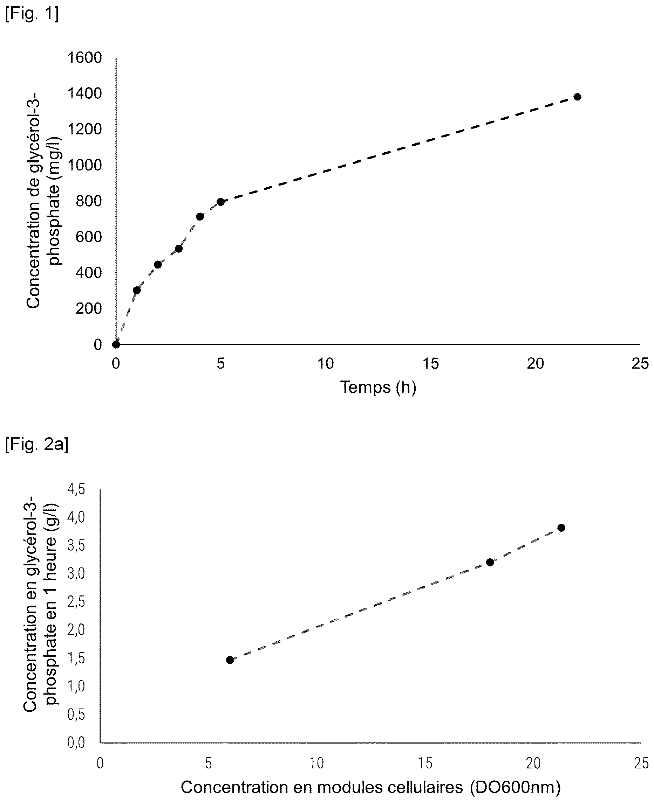

Les réactions de production de glycérol-3-phosphate sont réalisées dans des volumes de 2 ml de mélange réactionnel par resuspension du culot contenant une quantité équivalente de cellules Glpk et AckA de 2uDO dans un volume de 2 ml. Les tubes sont ensuite placés à 25°C sous agitation. Des échantillons sont réalisés sur le mélange réactionnel après l'ajout d'enzyme à 0 heure, et après 1 heure, 3 heures, 5 heures et 22 heures. Les résultats sont présentés à la Figure 1. Après 22 h de réaction, une production de 1,4 g/l glycérol-3-phosphate a été obtenue.

### Influence de la concentration cellulaire sur le rendement

Le même protocole a été répété avec une concentration en modules doublée. Une production de 12 g/l a été obtenue en 23 h, correspondant à un rendement de 81,5%, soit 37 fois plus que la production maximale obtenue par voie microbienne (Popp et coll. 2008). Ainsi, la concentration en cellules a une influence importante et pourra être optimisée.

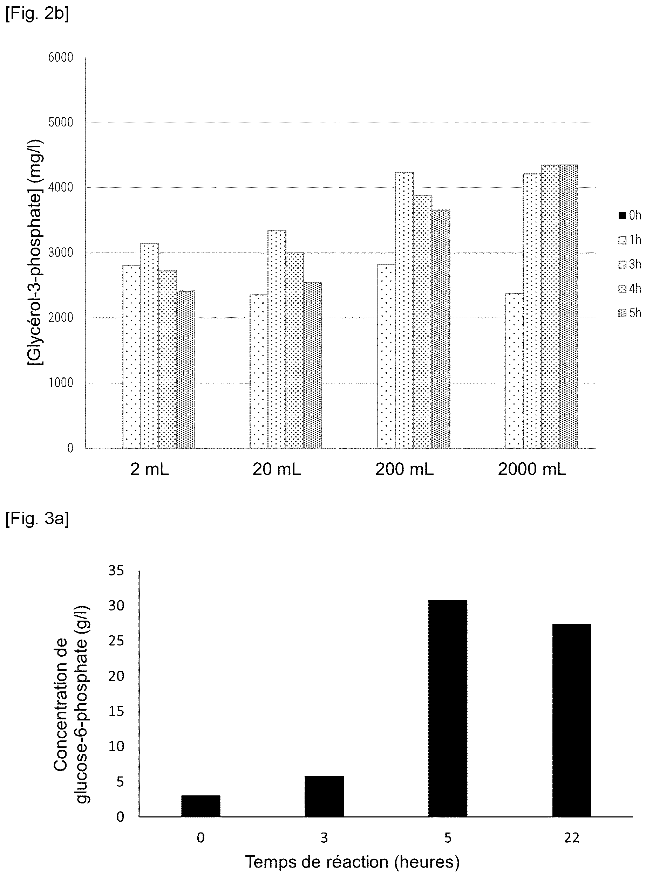

Influence de la concentration cellulaire sur la vitesse de réaction Afin d'évaluer l'effet de la quantité de cellules sur la vitesse de production, différentes concentrations ont été utilisées, dans les mêmes conditions expérimentales. Les concentrations en glycérol-3-phosphate obtenues en une heure montrent que la vitesse de la réaction est proportionnelle à la concentration en cellules (Figure 2a). Des productivités comprises en 1 g/l/h et 4 g/l/h peuvent donc être obtenues. Ces rendements indiquent qu'une montée en échelle du procédé selon l'invention est possible.

Cet exemple démontre la possibilité de produire des polyols phosphorylés par le procédé objet de l'invention. Celui-ci a permis de surmonter les obstacles liés à l'export et à la déphosphorylation par des phosphatases intracellulaires.

### Influence du volume réactionnel

Les procédés selon l'invention a vocation à produire des molécules à différentes échelles, du laboratoire jusqu'au niveau industriel. Afin d'évaluer l'effet de l'augmentation du volume du mélange réactionnel sur la production de glycérol-3-phosphate, la réaction a été réalisée en volumes de 2 ml, 20 ml, 200 ml et 2000 ml. Les modules utilisés sont comme précédemment BL21-Glk et BL21- AckA. Le mélange réactionnel est composé de :
Module 1 : 2 uDO/ml de cellules BL21-Glk
Module 2 : 2 uDO/ml de cellules BL21- AckA
Substrat : glycérol (25mM)
Cofacteur : ATP (5 mM)
Substrat complémentaire : phosphate d'acétyle (25 mM)
Solution tamponnée : HEPES (50 mM) et MgSO4 (15 mM)

Des prélèvements ont été effectués après l'ajout des biocatalyseurs cellulaires (modules) au mélange à 0 h, puis à 1 h, 3 h, 4 h et 5 h de réaction. Dans les conditions décrites, avec 25 mM de phosphate d'acétyle, la production maximale théorique de glycérol-phosphate est de 4,3 g/l. Les profils de cinétique de production observés à 2 ml et 20 ml sont similaires. La production maximale est de 3,3 g/l après 3 h avec un rendement de 76%. Lorsque le volume réactionnel est de 200 ml ou 2000 ml, 100% du rendement est atteint après 3 h. Ces résultats mettent en évidence le caractère transposable du procédé, pré-requis essentiel à une montée à l'échelle.

Et à 2000 ml le produit est stable. D'après ces résultats, la montée en échelle du procédé d'un facteur 1000 est tout à fait possible, et elle est même favorable à la production.

### EXEMPLE 1.2 : Production de glucose-6-phosphate à partir de glucose

Le glucose-6-phosphate est un point d'entrée de la voie de la glycolyse et de la voie des pentoses phosphates. Pour cette production, deux modalités ont été expérimentées, l'une avec des enzymes libres dans le périplasme, l'autre avec les mêmes enzymes ancrées à la membrane interne.

### Modalité avec enzymes libres

Les deux modules utilisés sont le module BL21-Glk exprimant la kinase Glk d'Escherichia coli, et le module de régénération de l'ATP BL21-AckA où l'acétate kinase d'Escherichia coli est exprimée. Les enzymes se trouvent à l'état libre dans le périplasme. Les deux modules sont cultivés et induits à l'IPTG en milieu TB, puis introduits dans le milieu mélange réactionnel à hauteur de 2 uDO dans 2 ml. Un échantillon de contrôle comprenant la souche BL21-WT est préparé. Le mélange réactionnel comprend :
Module 1 : 2 uDO/ml de cellules BL21-Glk
Module 2 : 2 uDO/ml de cellules BL21- AckA
Substrat : 300 mM de glucose
Cofacteur : 5 mM d'ATP
Substrat complémentaire : 125 mM de phosphate d'acétyle
Solution tamponnée : HEPES (50 mM) et MgSO4 (15 mM)

La Figure 3a montre que le glucose-6-phosphate est bien synthétisé, avec une concentration de 30,8 g/l obtenue dès 5 h de réaction, soit un rendement de 96%, limité par la quantité de phosphate d'acétyle (125 mM). Un plateau est donc atteint rapidement. Au bout de 22h, une diminution du glucose-6-phosphate est constatée, due à la dégradation de la molécule. Aucune production n'est observée avec la souche contrôle.

### Modalité avec enzymes ancrées

Les modules utilisés ici (BL21-nlpA-Glk et BL21-nlpA-ackA) expriment les deux mêmes enzymes (glucokinase Glk et acétate kinase AckA), mais celles-ci sont fusionnées au peptide d'ancrage nlpA, de sorte que les deux enzymes sont ancrées à la membrane interne du périplasme dans leur module respectif. Les modules sont cultivés et testés selon le même protocole que ci-dessus.

Le schéma réactionnel est le suivant :

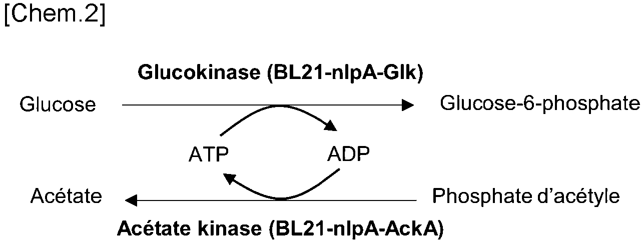

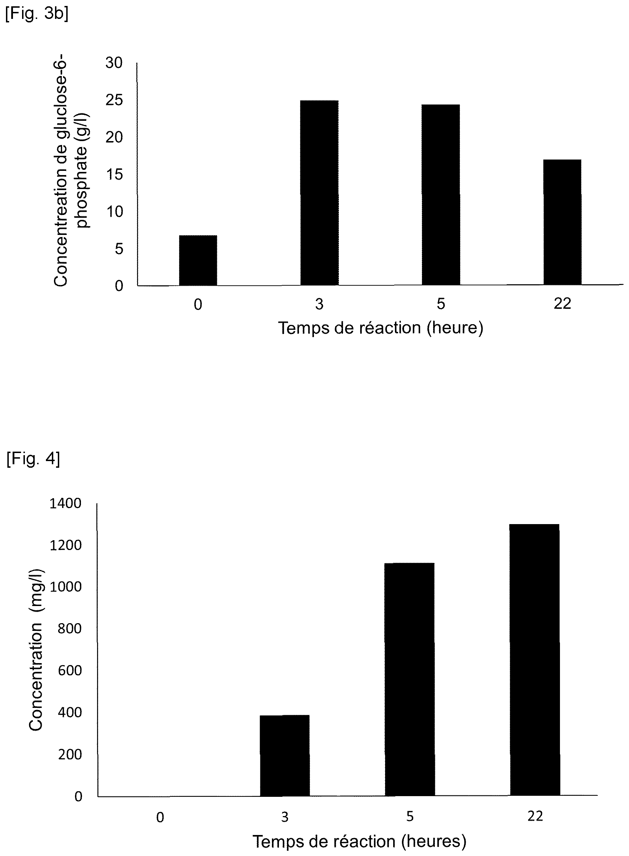

La Figure 3b montre une production de glucose-6-phosphate à une concentration de 24,8 g/l obtenue après 3 heures de réaction, soit un rendement de 76 %, limité par la quantité de phosphate d'acétyle (125 mM). Au bout de 22h, une diminution du glucose-6-phosphate est constatée, due à la dégradation de la molécule. Aucune production n'est observée avec la souche contrôle.

Conclusion : les réactions de production sont possibles lorsque les enzymes sont libres ou ancrées, dans cet exemple la production de glucose-6-phosphate à partir d'enzymes ancrées est plus rapide qu'à partir d'enzymes libres mais le rendement est plus faible. Le procédé selon l'invention peut être mis en œuvre avec des bactéries transformées générant des enzymes périplasmiques, qu'elles soient ancrées ou libres.

### EXEMPLE 1.3 : Production de fructose-1-phosphate à partir de fructose

Le fructose-1-phosphate est un sucre phosphorylé en position 1 à partir duquel on peut générer du glycéraldéhyde, un synthon d'intérêt. Sa synthèse par voie biologique n'a jamais été décrite.

Comme précédemment, deux modules sont nécessaires. L'un porte la séquence codant la fructokinase KhkC d'Homo sapiens qui est capable de phosphoryler des sucres en position 1 (BL21-KhkC). L'autre exprime l'acétate kinase d'Escherichia coli chargée de régénérer le cofacteur ATP (BL21-AckA déjà décrit).

Pour ce test, 4 uDO/ml de chaque module ont été apportés au mélange réactionnel, soit un total de 8 uDO dans 2 ml. Un échantillon de contrôle comprenant la souche BL21-WT est préparé. Le mélange réactionnel comprend :
Module 1 : 2 uDO/ml de cellules BL21- KhkC
Module 2 : 2 uDO/ml de cellules BL21- AckA
Substrat : 300 mM de fructose
Cofacteur : 5 mM d'ATP
Substrat complémentaire : 125 mM de phosphate d'acétyle
Solution tamponnée : HEPES (50 mM) et MgSO4 (5 mM)

Un échantillon de contrôle comprenant la souche BL21-WT (2uDO/mL autrement nommé DO₆₀₀ₙₘ = 2) est préparé.

Les résultats présentés à la Figure 4 montrent que la réaction est presque terminée au bout de 5 heures. Elle atteint un plateau après 22 heures avec une concentration de 1,3 g/L en fructose-1-phosphate. La réaction est limitée par le phosphate d'acétyle (125 mM). Aucune production n'est obtenue avec la souche contrôle.

Conclusion : Le procédé objet de l'invention est ainsi adapté à la production de sucres phosphorylés. En premier lieu, il est établi que la production de sucres phosphorylés par externalisation des réactions de phosphorylation dans le périplasme est possible et efficace. En second lieu, l'utilisation d'une même souche (BL21-AckA) dans plusieurs schémas réactionnels illustre la souplesse du procédé et son intérêt économique, en limitant le travail de génie biomoléculaires pour l'obtention des modules requis. Enfin, l'intérêt d'un module conçu pour la régénération du cofacteur est de pouvoir utiliser de faibles concentrations en ATP pour la production de molécules phosphorylées. Il est dès lors possible de concevoir des schémas réactionnels utilisant l'ATP naturellement présent en faible quantité dans le compartiment périplasmique.

### EXEMPLE 2 : Conservation et réutilisation des cellules

Dans le procédé selon l'invention, le recours à des modules mis en œuvre de manière découplée de la phase de culture cellulaires préalable, permet d'effectuer plusieurs cycles réactionnels avec la même biomasse et de conserver cette biomasse dans le temps.

Une production de glucose-6-phosphate a été conduite en réutilisant les cellules des modules BL21-GLK et BL21-AckA ayant déjà servi dans une production antérieure. A cet effet, une première production a été réalisée dans laquelle les cellules ont été mises en suspension dans 2 ml de mélange réactionnel (8 uDO pour chaque type cellulaire). Puis les cellules utilisées pour cette première production ont été séparées du surnageant, et les modules séparés par centrifugation. Les culots ont été récupérés et conservés à 4°C. Après quatre jours, les cellules ont été resuspendues dans de l'eau et introduites dans un nouveau volume du mélange réactionnel de 2 ml à hauteur de 8 uDO pour chaque type cellulaire.

Le mélange réactionnel utilisé pour les deux cycles comprend :
Module 1 : 8 uDO/ml de cellules BL21-GIK
Module 2 : 8 uDO/ml de cellules BL21-AckA
Substrat : 300 mM de glucose
Cofacteur : 5 mM d'ATP
Substrat complémentaire : 220 mM de phosphate d'acétyle
Solution tamponnée : HEPES (50 mM) et MgSO4 (5 mM)

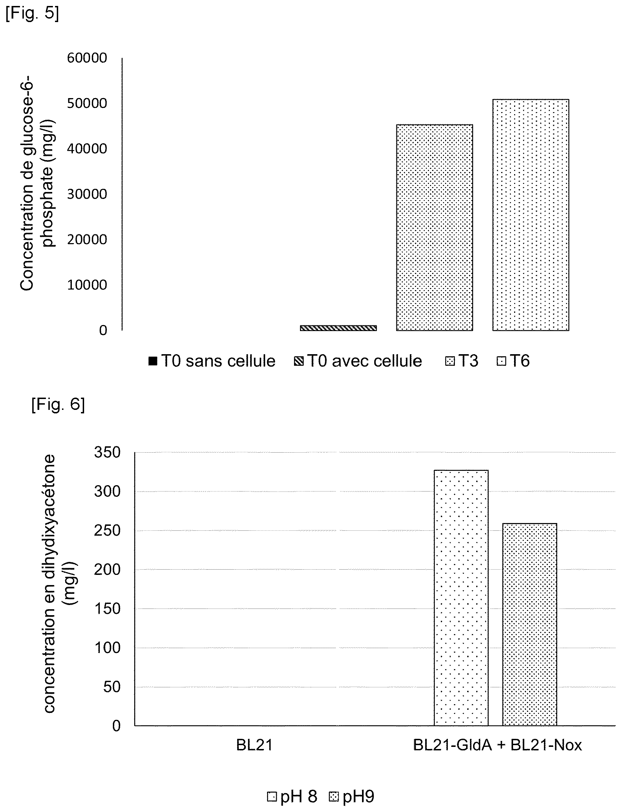

Les résultats présentés à la Figure 5 font apparaître une production de 50 g/l de glucose-6-phosphate en 6 heures. La réaction serait totale si la concentration finale obtenue était de 65 g/l. Le rendement de la réaction est donc de 75%. En réutilisant les cellules, il a été produit 1,6 fois plus de glucose-6-phosphate que lors de leur première utilisation où 30 g/l avaient été produits. Le procédé selon l'invention permet donc de conserver et d'utiliser plusieurs fois le même biocatalyseur sans perte d'efficacité, et de réduire ainsi le coût lié à la production de biomasse.

### EXEMPLE 3 : Production de dihydroxyacétone à partir de glycérol

Le dihydroxyacétone (DHA) est un agent tannant très utilisé dans l'industrie cosmétique. Le procédé selon l'invention a été mis en œuvre pour produire du DHA. Il inclut le recyclage des cofacteurs d'oxydo-réduction impliqués dans la réaction.

Pour ce faire, deux modules ont été construits. Une souche d'Escherichia coli a été transformée pour exprimer dans son périplasme une glycérol déshydrogénase d'Escherichia coli catalysant la conversion du glycérol en DHA (premier module BL21-GldA). Cette réaction consomme un cofacteur, à savoir du NAD+ et forme du NADH en tant que coproduit. Un second module (module BL21-Nox) a été construit en transformant une souche d'Escherichia coli pour qu'elle exprime dans son périplasme la NADH réductase (NOX) de Lactobacillus sanfranciscensis. Cette enzyme catalyse une réaction utilisant de l'oxygène (O₂) et du NADH pour former de l'eau (H₂O) et du NAD+, lequel est ainsi régénéré pour intervenir dans la première réaction. Le plasmide pET26b portant le gène nox de L.sanfransiscensis qui code pour la NADH oxydase NOX a été commandé chez Genecript et transformé dans souche BL21-WT.

Le schéma réactionnel est le suivant :

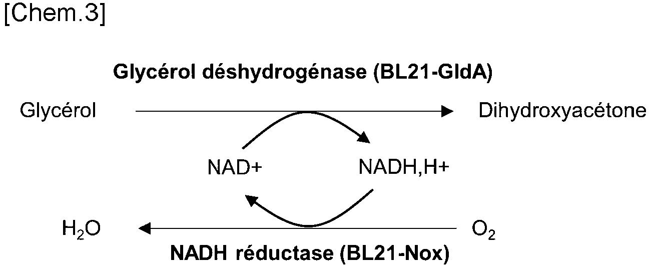

Les deux modules sont cultivés et induits à l'IPTG en milieu TB avant d'être récupérés pour effectuer la production de DHA. Le mélange réactionnel comprend :
Module 1 : 4 uDO/ml de cellules BL21-GIdA
Module 2 : 4 uDO/ml de cellules BL21-Nox
Substrat : 20 mM de glycérol
Cofacteur : 5 mM de NAD+
Solution tamponnée : de 100 mM (Tris pH 8 ou Gly pH 9).

Des témoins négatifs sans substrat ont été réalisés. De plus, des témoins négatifs où chaque souche est remplacée par des cellules BL21(DE3) n'exprimant pas les enzymes GldA ou NOX ont été réalisés. Le pH optimal de GldA étant à 9 tandis que celui de NOX est à 7,5, des essais ont été réalisés aux pH 8 et 9 afin de trouver le meilleur compromis pour le fonctionnement de la réaction. Les mesures ont été réalisées après 5 heures et sont présentées en Figure 6.

Une production de DHA est obtenue en présence des deux modules, à pH 8 et à pH 9, alors qu'aucune production n'apparaît dans le contrôle sauvage. Une quantité de DHA de 260 mg/l à pH 8 et de 330 mg/l à pH 9 a été obtenue, démontrant la faisabilité du schéma réactionnel proposé.

### EXEMPLE 4 : Production de glycolaldéhyde à partir de glyoxal

Le glycolaldéhyde est une molécule très réactive présente dans le métabolisme cellulaire et pouvant être employée comme la plupart des aldéhydes en tant que molécule plateforme. Néanmoins, son prix est trop élevé pour une utilisation industrielle. Du fait même de leur réactivité qui les rend toxiques pour les cellules et instables, les aldéhydes ne sont que rarement produits par voie microbienne. Leur export est en outre compliqué par leur capacité à réagir avec les protéines et les lipides de la membrane. Le glycolaldéhyde pourrait être obtenu par réduction du glyoxal qui est peu coûteux, mais nettement toxique.

Pour ce faire, deux modules ont été construits. Une souche d'Escherichia coli a été transformée pour exprimer dans son périplasme la glyoxal réductase YvgN de Bacillus subtilis, catalysant la conversion du glyoxal en glycolaldéhyde (premier module BL21-YvgN). Cette réaction consomme un cofacteur, à savoir du NADPH, et forme du NADP en tant que coproduit (Dudek et coll., 2013). Un second module (BL21-PtxD) a été construit en transformant une souche d'Escherichia coli pour qu'elle exprime dans son périplasme la phosphite déshydrogénase PtxD qui est une NADP réductase. Le gène ptxD de Pseudomonas stutzeri qui code pour cette enzyme comprend 18 mutations assurant une efficacité catalytique augmentée, une stabilité à 37°C et la prise en charge des deux substrats NAD et NADP. Le gène yvgn de Bacillus subtilis code pour la glyoxal réductase (GR). La version sauvage a été utilisée. Les plasmides d'expression pET22b-PtxD mutant et pET26b-YvgN ont été commandés à Genescript. Le gène ptxD a été commandé codon optimisé pour l'expression dans E. coli. Les plasmides pET22b et pET26b possèdent la séquence signal pelB responsable de l'export des protéines dans le périplasme, pET22b est résistante à l'ampicilline, pET26b à la kanamycine. Ces plasmides ont ensuite été transformés dans BL21-WT pour donner les modules BL21-YvgN et BL21-PtxD. Le schéma réactionnel est le suivant :

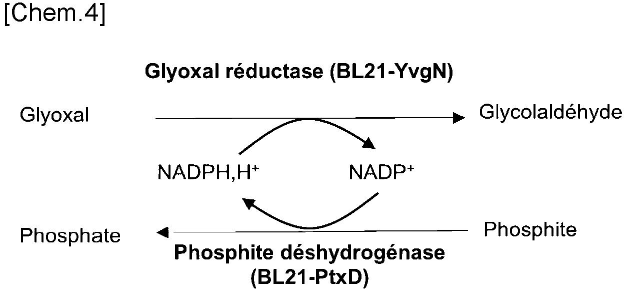

Le mélange réactionnel comprend :
Module 1 : 1 uDO/ml de cellules BL21-YvgN.
Module 2 : 1 uDO/ml de cellules BL21-PtxD (sauf pour essai préalable)
Substrat : 10 mM de glyoxal
Cofacteur : 0,4 mM NADPH
Substrat complémentaire : 10 mM phosphite
Solution tamponnée : 50 mM d'HEPES pH 7,5 et 10 mM de KCl.

Dans un essai préalable, la production d'aldéhyde par la glyoxal réductase périplasmique a été réalisée seule, sans système de régénération. Dans un deuxième temps, les réactions sont initiées par l'ajout au mélange réactionnel des cellules induites BL21-PtxD à une concentration de 1 uDO, et de phosphite à 10 mM afin de régénérer le NADP produit en NADPH. Les réactions sont placées à une température de 37°C sous une agitation de 200 rpm. Les mesures sont réalisées à 0 heure, 1 heure, 3 et 5 heures.

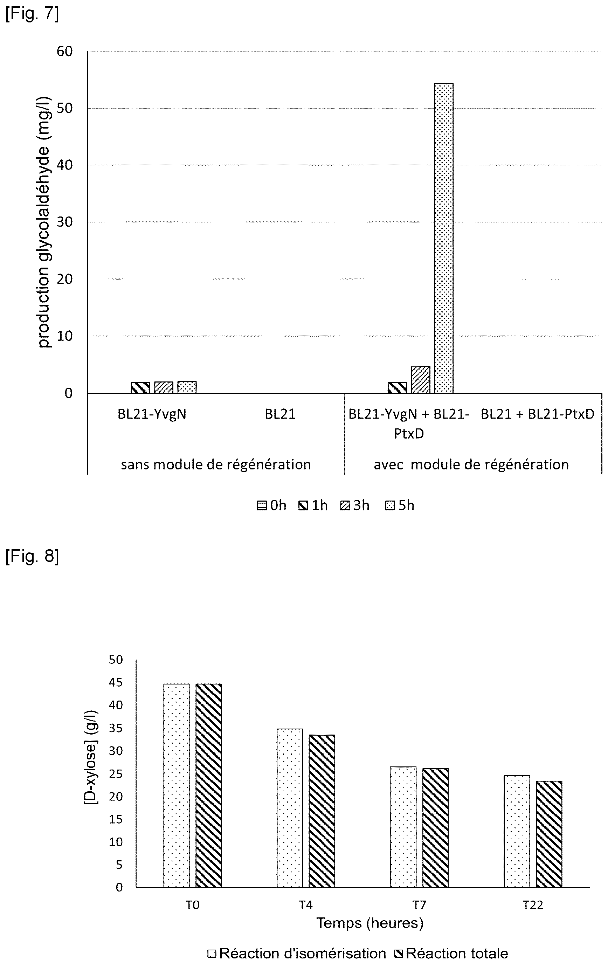

Les résultats portés dans la Figure 7 montrent que du glycolaldéhyde est détecté dès 1 h de réaction avec et sans système de régénération, mais la production atteint déjà un palier en l'absence de PtxD du fait de l'épuisement du NADPH. Au contraire, en présence du module BL21-PtxD, la réaction se poursuit grâce à la régénération du NADPH. Après 5 h, la production glycolaldéhyde est 25 fois plus élevée, pour un rendement de 37 %.

Les modules BL21-YvgN et BL21-PtxD produisent donc efficacement du glycolaldéhyde à partir de glyoxal dans le compartiment périplasmique, sans effet néfaste sur les cellules bactériennes. Ceci démontre qu'il est possible par le procédé selon l'invention, de produire des composés aldéhydiques à partir d'un précurseur toxique.

### EXEMPLE 5 : Production d'un alcool à partir d'un acide aminé

Un schéma réactionnel a été élaboré qui permet de produire du 2-phényléthanol à partir de la L-phénylalanine. Ce schéma réactionnel repose sur l'utilisation de quatre modules distincts. Trois modules ont été construits qui expriment respectivement les gènes ARO8, ARO10, ADH5 de S. cerevisiae codant l'un pour la L-phénylalanine oxoglutarate aminotransférase, le second pour la phénylpyruvate carboxylase et le troisième pour la 2-phényléthanol déshydrogénase. Un quatrième module exprimant la phosphite déshydrogénase PtxD est conçu pour la régénération du NADH.

Le schéma réactionnel est le suivant :

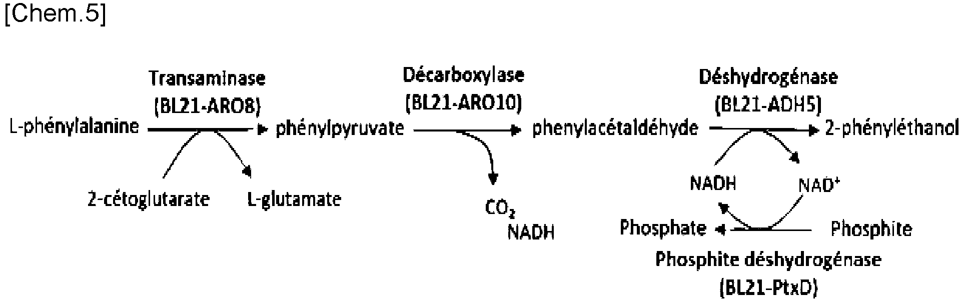

C'est ici la troisième réaction produisant le composé d'intérêt qui donne lieu à un couplage avec une régénération du cofacteur NADH à partir du coproduit NAD+.

Le mélange réactionnel est le suivant :
Module 1 : 5 DO / ml de cellules BL21-ARO8 Module 2 : 5 D0₆₀₀ₙₘ / ml de cellules BL21-ARO10
Module 3 : 5 D0₆₀₀ₙₘ / ml de cellules BL21-ADH5
Module 4 : 5 D0₆₀₀ₙₘ / ml de cellules BL21-PtxD
Substrat : L-phénylalanine (10 mM)
Cofacteurs : 2-cétoglutarate (10 mM), MgCl2 (5 mM), ZnCl2 (5 mM), Thiamine (0,2 mM), pyridoxal (0,1 mM), NADH (1 mM)
Substrats complémentaires : phosphite (100 mM)
Solution tamponnée : HEPES pH 7 (100 mM).

Les réactions de 2 mL ont été placées à 33 °C, agitées à 200 rpm pendant 5h. Des prélèvements ont été effectués à 0, 1, 3 et 5 h.

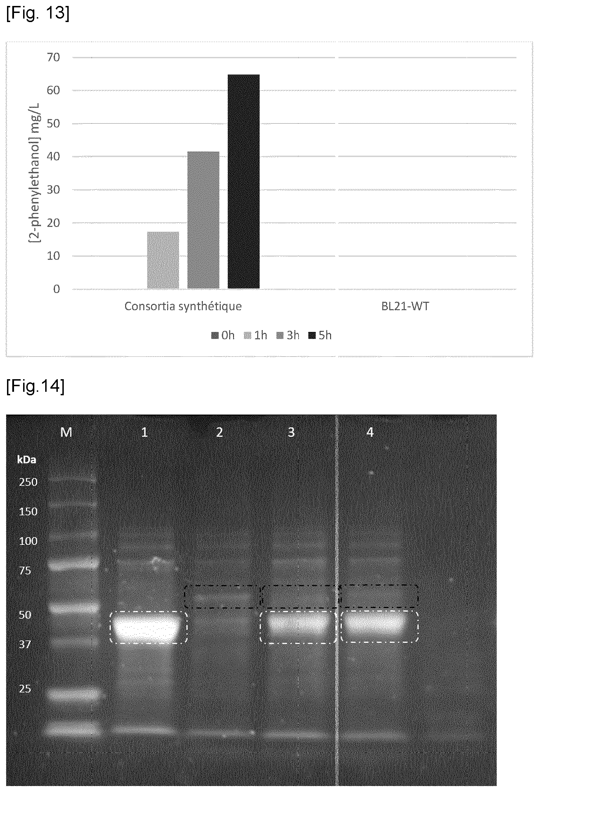

Le consortia synthétique composé des quatre biocatalyseurs bactériens BL21-ARO8, BL21-ARO10, BL21-ADH5 et BL21-PtxD a été utilisé pour produire du 2-phényléthanol à partir de la L-phénylalanine. L'efficacité de ce consortia a été testé et validé en mesurant l'apparition de 2-phenylethanol par HPLC. **[****Fig.13****]** Les résultats ont montré que la synthèse de 65 mg/L de 2-phényléthanol a été obtenue en 5 heures de réaction.

### EXEMPLE 6 : Production d'isomères de sucres rares

La production de sucres rares se fait principalement par isomérisation d'un sucre commun. La difficulté provient de ce que l'isomérisation est une réaction réversible. L'objectif est ici de limiter la réaction inverse en transformant le sucre rare formé en un nouveau composé, par exemple en sucre phosphorylé, laquelle est ensuite déphosphorylée pour obtenir l'isomère d'intérêt.

Dans cet exemple, une première réaction isomérise un sucre simple, le D-xylose, en un sucre rare, le D-xylulose. Une deuxième réaction phosphoryle le D-xylulose en D-xylulose-5-phosphate, en présence d'ATP agissant comme cofacteur. Le schéma réactionnel se poursuit par deux réactions en aval, à savoir d'une part une réaction consommant l'ADP formé et du phosphate d'acétyle pour régénérer de l'ATP, et d'autre part une réaction de déphosphorylation du D-xylulose-5-phosphate, redonnant du D-xylulose.

Quatre modules différents sont utilisés.
- Le premier module (BL21-XylA) est une souche d'Escherichia coli transformée pour exprimer dans son périplasme la xylose isomérase XylA présente chez E. coli.
- Le deuxième module (BL21-XyIB) est une souche d'Escherichia coli transformée pour exprimer une kinase, XylB de E. coli, permettant de phosphoryler le xylulose en xylulose-5P en consommant une molécule d'ATP.
- Le module BL21-AckA déjà vu plus haut est utilisé pour régénérer de l'ATP.
- Le quatrième module (BL21-AphA) est une souche d'Escherichia coli transformée pour exprimer dans son périplasme la phosphatase à large spectre AphA d'E. coli (Passariello et coll., 2006).

Le schéma réactionnel complet est présenté ci-dessous.

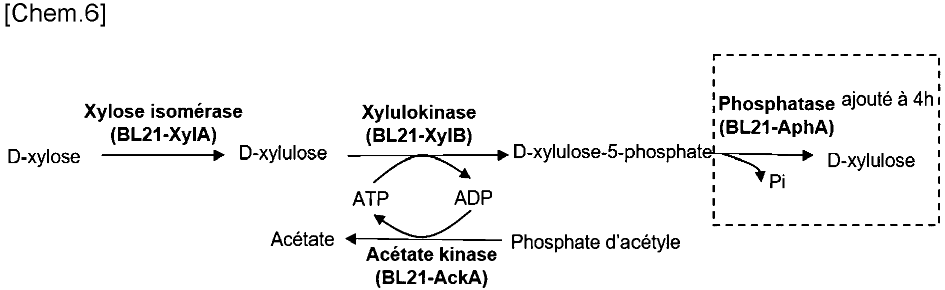

Les modules sont cultivés et induits à l'IPTG en milieu TB avant d'être récupérés pour effectuer la production de D-xylulose. Le mélange réactionnel comprend :
Module 1 :8 uDO/ml de cellules BL21-XylA
Module 2 : 8 uDO/ml de cellules BL21-XyIB
Module 3 : 8 uDO/ml de cellules BL21-AckA
Module 4 : 8 uDO/ml de cellules BL21-AphA
Substrat : D-xylose (300 mM)
Cofacteur : d'ATP (5 mM)
Substrat complémentaire : phosphate d'acétyle (125 mM)
Solution tamponnée : HEPES pH 7,5 (50 mM), MgSO4 (5 mM), MnSO4 (3 mM)

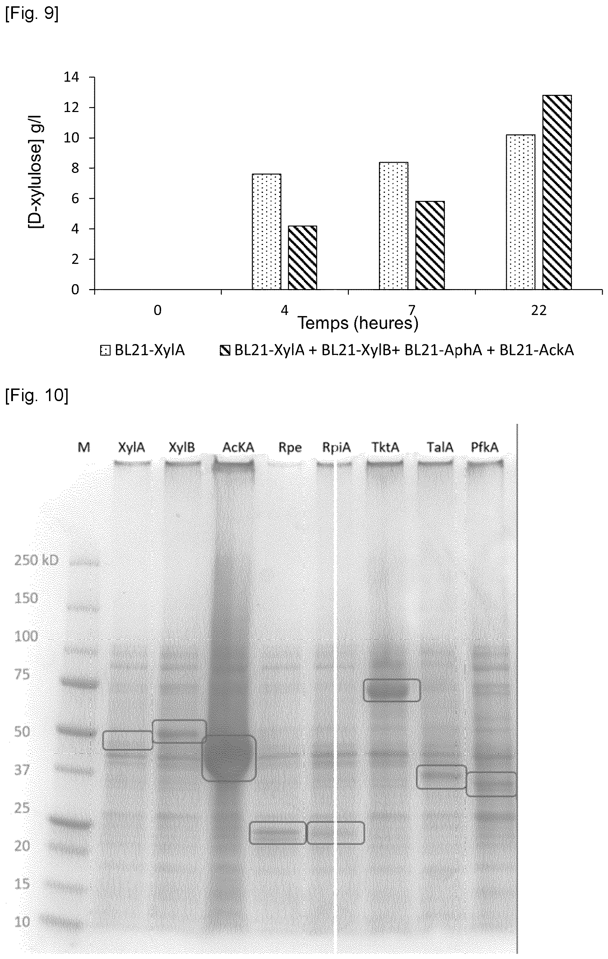

Le processus est initié avec les modules 1, 2 et 3 exprimant chacun une enzyme dans le mélange réactionnel. Les tubes sont placés à une température de 37°C sous agitation à 200 rpm. L'ajout du module 4 exprimant la phosphatase AphA se fait 4 heures après le début de la réaction et le pH est ajusté à 6. Le suivi de la réaction est réalisé à 7 heures puis à 22 heures. En parallèle, une réaction est menée avec le module BL21-XylA seul, afin d'évaluer la réaction d'isomérisation simple. L'évolution des concentrations de D-xylose (Figure 8) et de D-xylulose (Figure 9) au cours de la réaction a été suivie par HPIC.

La Figure 8 montre que le substrat D-xylose est consommé dans des quantités similaires dans les deux modalités. Par contre, après 4 heures, la production de D-xylulose est deux fois moindre pour la réaction complète que pour la réaction d'isomérisation seule (Figure 9). Cette différence s'explique par la consommation du D-xylulose du fait de la production de D-xylulose-5P dans le schéma complet. Sa présence n'est pas détectable par la méthode de dosage de D-xylulose, mais a été confirmée par spectrométrie de masse.

Le module 4 portant la phosphatase AphA, ajouté au mélange réactionnel à T=4 h, catalyse la déphosphorylation du D-xylulose-5P en D-xylulose. Ainsi, 3 heures et 18 heures après l'introduction de la phosphatase, une augmentation respectivement de 2 g/m et 8 g/l de D-xylulose est observée. La quantité de D-xylulose de la réaction complète (12,8 g/l) dépasse celle de la réaction d'isomérisation seule (10,2 g/l), soit un gain significatif de 2,6 g/l.

Ceci s'explique par le fait qu'au cours de la réaction d'isomérisation, un équilibre se crée entre les deux espèces D-xylose et D-xylulose, de sorte qu'il ne peut y avoir de conversion totale du D-xylose. La présence du module kinase BL21-XylB dans le mélange réactionnel permet de piéger le D-xylulose sous forme phosphorylée, qui alors ne peut plus être converti en D-xylose. Le D-xylulose-5P s'accumule dans le mélange réactionnel, et l'ajout de la phosphatase BL21-Apha permet de récupérer du D-xylulose en quantité accrue comparée à une simple isomérisation. En tirant ainsi l'isomérisation vers une forme phosphorylée du sucre rare, on obtient un taux de conversion plus élevé.

Ces résultats montrent en premier lieu que l'isomérisation, la phosphorylation et la déphosphorylation par les modules BL21-XylA, BL21-XylB et BL21-AphA sont possibles. Ils attestent aussi que fait que quatre modules différents ont fonctionné ensemble pour réaliser un schéma réactionnel complexe, incluant un mécanisme de régénération de l'ATP. L'utilisation synergique et séquentielle de toutes sortes de modules dans le cadre du procédé objet de l'invention est ainsi validée.

### EXEMPLE 7 : Production de D-fructose-1,6-bisphosphate partir de D-xylose

La conversion du D-xylose en D-fructose-1,6-bisphosphate fait partie de la voie des pentoses phosphate qui est la voie d'assimilation naturelle du D-xylose dans le cytoplasme. Cette conversion a été réalisée par un schéma réactionnel mettant en œuvre neuf réactions réalisées par huit modules différents.

Les huit modules utilisés sont construits à partir d'une souche d'Escherichia coli, chacune transformée pour exprimer dans son périplasme une enzyme présente chez E. coli :
1 - la xylose isomérase XylA, qui isomérise le D-xylose en D-xylulose
2 - la xylulose kinase XylB, qui phosphoryle le D-xylulose en D-xylulose-5P
3 - l'acétate kinase AckA, qui régénère l'ATP à partir d'ATP et de phospha d'acétyle
4 - la ribulose-phosphate-3-épimérase Rpe, qui transforme le D-xylulose-5P en D-ribulose-5P
5 - la ribose-5-phosphate isomérase RpiA, qui produit le D-ribose-5P à partir du D-ribulose -5P
6 - la transcétolase Tkt qui transforme le D-xylulose-5P et le D-ribose-5P en D-glycéraldéhyde-3P et D-sédoheptulose-7P
7 - la transaldolase Tal qui transforme le D-glycéraldéhyde-3P et le D-sédoheptulose-7P en D-fructose-5P et D-érythrose-4P
8 - la 6-phosphofructokinase 1 PfkA qui transforme le D-fructose-5P en D-fructose-1,6bP

Le schéma réactionnel complet est présenté ci-dessous.

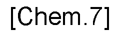

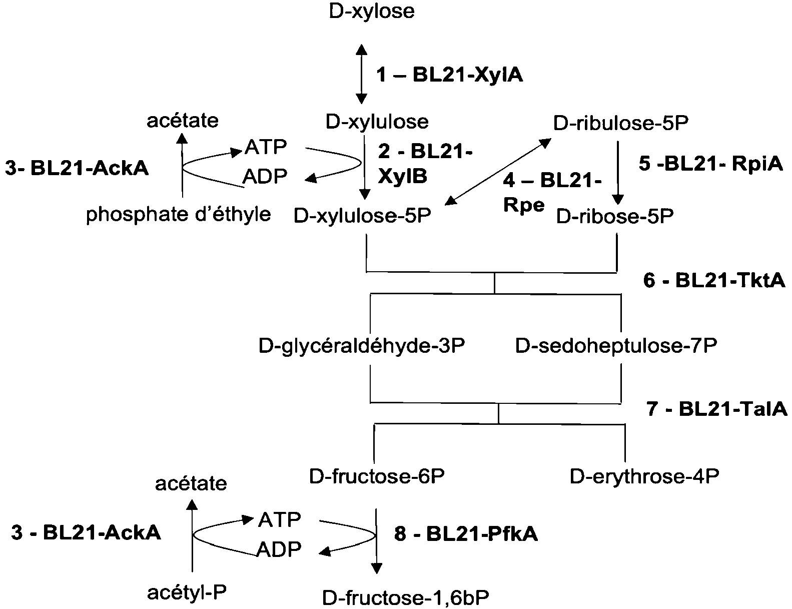

La présence des enzymes dans le périplasme des huit modules a été vérifiée en récupérant la fraction périplasmique par choc osmotique pour chacune des souches. La taille des enzymes et la quantité de cellules utilisées pour le choc osmotique (DO600nm multipliée par le volume de culture) sont données dans le Tableau 4.

**[Tableau 4] : Poids moléculaire en kilodalton des protéines XylA, XylB, AckA, Rpe, RpiA, TktA, TalA et PfkA et densité optique de la culture utilisée pour le choc osmotique**

| **Protéine** | **XylA** | **XylB** | **AcK** | **Rpe** | **RpiA** | **TktA** | **TalA** | **PfkA** |
|---|---|---|---|---|---|---|---|---|
| Taille (kDa) | 44-49 | 52 | 43,3 | 24 | 22-27 | 73 | 35 | 35 |
| DO600nm | 2,84 | 2,04 | 9,2 | 2,38 | 1,86 | 4,46 | 1,34 | 8,74 |

Les résultats de l'analyse par gel SPS-PAGE sont donnés en Figure 10. Ils montrent que l'expression dans le périplasme des enzymes attendues pour les 8 modules est validée.

Les modules ont été cultivés et induits à l'IPTG en milieu TB avant d'être récupérés pour effectuer la production de D-fructose-1,6bP dans le mélange réactionnel comprenant :
Module 1 : 8 uDO/ml de cellules BL21-XylA
Module 2 : 8 uDO/ml de cellules BL21- XylB
Module 3 : 8 uDO/ml de cellules BL21- AckA
Module 4 : 8 uDO/ml de cellules BL21- Rpe
Module 5 : 8 uDO/ml de cellules BL21- RpiA
Module 6 : 8 uDO/ml de cellules BL21- Tkt
Module 7 : 8 uDO/ml de cellules BL21- Tal
Module 8 : 8 uDO/ml de cellules BL21- PfkA
Substrat : 100 mM de D-xylose
Cofacteur : 3 mM d'ATP
Substrat complémentaire : 100 mM phosphate d'acétyle
Solution tamponnée : HEPES pH 7,5 (50 mM), MgSO4 (20 mM), MnSO4 (1 mM) et thiamine pyrophosphate (1 mM) CoCl2 (0,1 mM)

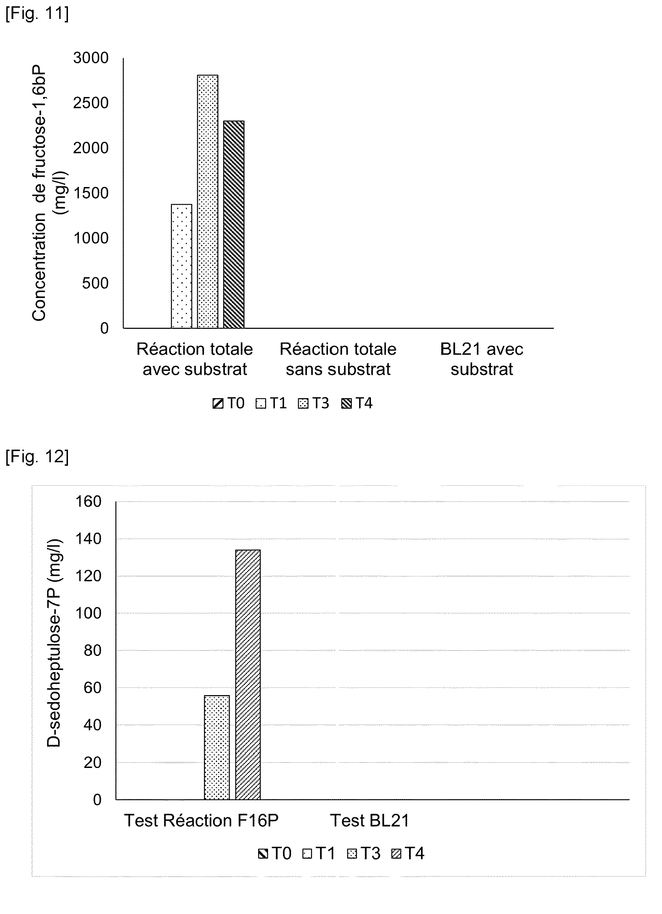

Le dosage du D-fructose-1,6bP a été réalisé immédiatement après ajout des modules au mélange réactionnel, après 1 heure, 3 heures et 4 heures. Deux contrôles ont été effectués, l'un sans substrat et l'autre sans module (les huit modules sont remplacés par des cellules BL21 WT. Les résultats sont présentés dans la Figure 11. Après 1 h de réaction, la production de D-fructose-1,6-bisphosphate est de 1,3 g/l et atteint un maximum de 2,8 g/l en 3 h. Aucune production de D-fructose-1,6-bisphosphate n'est mesurée dans les deux réactions de contrôle.

Le D-sédoheptulose-7-phosphate a également été dosé (Figure 12). En effet, les modules porteurs de XylB et PfkA catalysent les seules réactions irréversibles du schéma réactionnel, et le D-sédoheptulose-7-phosphate est au milieu de la chaine réactionnelle composée de réactions réversibles dont les produits s'équilibrent entre eux. Le D-sedoheptulose-7-phosphate a été dosé immédiatement après ajout des modules au mélange réactionnel, après 1 heure, 3 heures et 4 heures. Il est de à 130 mg/l après 4 heures dans la réaction complète et est absent des échantillons de contrôle. Les autres composés intermédiaires sont difficilement détectables avec les méthodes analytiques utilisées, mais le niveau de D-sedoheptulose-7P est représentatif de leur concentration, qui est donc similaire. La production de D-fructose-1,6-bisphosphate selon un schéma réactionnel complexe a donc été réalisée avec succès.

Ces résultats démontrent que le procédé objet de l'invention reposant sur l'externalisation de réactions fonctionne efficacement, y compris pour une chaine longue et ramifiée, impliquant de nombreux modules. Il est en outre particulièrement intéressant d'avoir pu reconstituer ainsi une voie métabolique complexe naturellement cytoplasmique.

### EXEMPLE 8 : Effet d'un consortium de 2 bactéries exprimant chacune 1 enzyme, comparativement à une bactérie exprimant 2 enzymes, sur la production d'un produit (G3P)

La production de glycérol-3-phosphate repose sur l'activité catalytique de la glycérol kinase et de l'acétate kinase. Afin de démontrer l'intérêt de l'utilisation d'un consortia synthétique (2 bactéries exprimant chacune une enzyme différente), comparativement à une bactérie exprimant les 2 enzymes, nous avons créé un biocatalyseur de type whole-cell co-exprimant la glycérol kinase GlpK *d'Escherichia coli* et l'acétate kinase AckA d'*Escherichia coli* dans son périplasme. Pour ce faire, un plasmide pETDuet-pelB-glpK-pelB-ackA contenant deux sites de clonage sous le contrôle de deux promoteurs T7 a été construit et transformé dans *E.coli,* Ce biocatalyseur est appelé ici BL21-Glk-AckA. Le design du vecteur pETDuet a été réalisé de manière à ce que les cassettes d'expression du pETDuet soient identiques à celles du pET22b. Ainsi la comparaison des souches BL21-AckA, BL21-GlpK et BL21-GlpK-AckA a pour objet l'effet de la co-expression sans influence du promoteur, RBS ou peptide signal.

Les fractions périplasmiques des souches BL21-AckA et BL21-AckA-GlpK ont été récupérées après un choc osmotique à partir de 2 mL de suspension cellulaire concentrée à 2 de D0₆₀₀ₙₘ pour chacune des souches. Nous observons que la bande d'AckA à 43 kDa est d'une intensité au moins deux fois plus faible dans la souche BL21-AckA-GlpK comparé à la souche BL21-AckA. Ce résultat met en évidence un effet négatif de la co-expression de deux enzymes dans la même bactérie sur la production de l'enzyme AckA dans le périplasme du biocatalyseur BL21-AckA-GlpK (1 bactérie exprimant 2 enzymes). En comparant les bandes à 56 kDa de GlpK des souches BL21-GlpK et BL21-GlpK-AckA, nous observons également un effet négatif de la co-expression des 2 enzymes dans la même bactérie sur la production périplasmique de GlpK.

Enfin, nous avons extrait les fractions périplasmiques d'un mélange de BL21-AckA et BL21-GIpK (consortium de bactéries selon l'invention), où chacune des souches était à une concentration de 1 de D0₆₀₀ₙₘ condition se rapprochant des conditions standards de co-culture. Les bandes AckA et GlpK sont plus marquées dans la co-culture (consortium selon l'invention), comparativement à la fraction périplasmique extraite du biocatalyseur co-exprimant AckA et GlpK (1 bactérie exprimant 2 enzymes). **[****Fig.14****]** Ces résultats montrent que l'utilisation d'un consortium de bactéries permet d'obtenir une meilleure expression de chaque enzyme, comparativement à l'utilisation d'une même bactérie co-exprimant les 2 enzymes.

Nous observons par ailleurs que le peptide signal pelB permet une meilleure production de l'enzyme AckA que de l'enzyme GlpK dans le périplasme des souches BL21-AckA et BL21-GIpK respectivement. Cette observation n'est pas surprenante car l'efficacité des peptides signal dépend de la protéine cible. Il est possible de moduler la quantité de BL21-GlpK et BL21-AckA dans le mélange réactionnel de production de glycérol-3P pour maximiser l'efficacité de la réaction en augmentant la quantité de BL21-GIpK pour pallier à la faible efficacité de PelB. Cette stratégie permet d'améliorer facilement la production de glycérol-3P ce qui est avantageux pour une utilisation industrielle. En comparaison le biocatalyseur BL21-GlpK-AckA co-exprimant les enzymes GlpK et AckA n'offre pas cette modularité. Il faudrait reconstruire chacune des cassettes d'expression (promoteur et/ou RBS et/ou peptide signal) pour moduler l'expression de GlpK et AckA ; ce qui est plus complexe et chronophage.

Nous avons également comparé la production de G3P par le biocatalyseur BL21-GlpK-AckA (1 bactérie exprimant 2 enzymes) et le consortia synthétique BL21-GIpK et BL21-AckA (selon l'invention). Le mélange réactionnel est composé de :
Module(s) : cellules BL21-glpK et/ou BL21-ackA et/ou BL21-glpK-ackA (D0_{600nm totale} = 2)
Substrat : 160 mM de glycérol
Cofacteurs : 5mM d'ATP, 15 mM de MgSO4
Substrat complémentaire : 220 mM de phosphate d'acétyle
Solution tamponnée : 50mM de tampon HEPES à pH 7,5

Les réactions de 5 ml sont placées à une température de 37°C à une agitation de 200 rpm et suivie sur 24 heures. Des prélèvements de 200µL sont effectués à T0h, T0,5h, T1h, T2h, T3h, T5h et T24h.

Le biocatalyseur BL21-GlpK-AckA est capable de produire 2,1 grammes de G3P par litre en 24 heures. Cependant, en utilisant un mélange de deux types de biocatalyseurs - BL21-GlpK et BL21-AckA dans des proportions égales (50% de chaque), il est possible de produire 5 grammes de G3P par litre en 24 heures, soit une production plus de 2 fois plus importante que dans le système 1 bactérie co-exprimant 2 enzymes. Et en ajustant les proportions des deux types de biocatalyseurs, il est possible d'optimiser encore la production de G3P. En effet, en utilisant un mélange de 92,5% de BL21-GlpK et 7,5% de BL21-AckA, la production de G3P atteint 10 grammes par litre en seulement 5 heures. **[****Fig. 15****].** Ces résultats montrent qu'en modulant la proportion des biocatalyseurs BL21-GlpK et BL21-AckA, il est possible d'améliorer considérablement la bioproduction de G3P. C'est un avantage considérable de l'utilisation de consortiums selon l'invention de pouvoir moduler facilement les proportions de chaque biocatalyseur, et ce, de façon bien plus facile et fiable que via l'utilisation d'ingénierie génétique complexe au sein d'une même bactérie co-exprimant plusieurs enzymes.

### EXEMPLE 9 : Production à partir d'ATP extracellulaire

La purine adénosine 5'-triphosphate (ATP) n'est pas seulement un transporteur universel d'énergie intracellulaire, elle joue également un rôle important en tant que molécule de signalisation extracellulaire. L'ATP est bien connue en tant que messager pour les communications intercellulaires chez les organismes multicellulaires, mais des organismes unicellulaires phylogénétiquement plus anciens tels que la levure ou les bactéries utilisent également l'ATP en tant que molécule de signalisation extracellulaire. Cependant, les mécanismes de sécrétion de l'ATP par les bactéries et ses implications extracellulaires doivent encore être élucidés.( Spari D et Beldi G, 2020).

Alvarez et son équipe ont montré que dans E. coli DH5α, l'ATP est rapidement hydrolysé dans l'espace périplasmique. Il a été déterminé que la concentration d'ATP extracellulaire à l'état stationnaire dans l'espace périplasmique est de 24 ± 3 µM/10¹⁰ bactéries. (Alvarez et al., 2017).

Dans le contexte de la production de molécules phosphorylées, telles que le glucose-6-phosphate , l'ATP est un co-facteur essentiel pour la phosphorylation. Cependant, l'ATP est une molécule coûteuse à produire et doit souvent être régénérée à partir de molécules moins coûteuses, telles que l'ADP.

L'utilisation d'un module de régénération de l'ATP tel que BL21-AckA permet de réutiliser les traces d'ATP présentes dans le périplasme pour la production de molécules phosphorylées. Cela permet de réduire les coûts de production et d'utiliser efficacement les ressources naturelles de la cellule.

Des productions de G6P ont ainsi pu être réalisées selon le procédé de l'invention, sans ajout d'ATP commercial.

Le premier mélange réactionnel comprend :
Module 1 : 2 uDO/ml de cellules BL21-Glk
Module 2 : 2 uDO/ml de cellules BL21- AckA
Substrat : 300 mM de glucose
Substrat complémentaire : 250mM de phosphate d'acétyle
Solution tamponnée : HEPES (50 mM) et MgSO4 (15 mM). Lorsque la concentration cellulaire de chaque biocatalyseur est de 2 DO600nm/ml, il est possible de produire 4 g/L de glucose-6-phosphate sans ajout d'ATP. Cela suggère que la production d'ATP est suffisante pour soutenir la production de glucose-6P à cette densité cellulaire.

Le nombre de cellules d'Escherichia coli pour 1 DO600nm dépend de nombreux facteurs, notamment les conditions de croissance, le milieu de culture et la souche bactérienne utilisée. Cependant, il existe une corrélation empirique entre la densité optique à 600 nm et le nombre de cellules bactériennes. En général, pour E. coli, la densité optique à 600 nm de 1 correspond à une concentration cellulaire d'environ 8.10⁸ cellules/ml (https://www.agilent.com/store/biocalculators/calcODBacterial.jsp). Dans les conditions de la **figure 16** où chaque biocatalyseur est présent à une densité optique 600 nm de 2 / ml il y aurait 3,2.10⁹ cellules/mL. En considérant la concentration d'ATP 24 ± 3 µM/10¹⁰ bactéries fournie par Alvarez et son équipe, la concentration d'ATP dans l'essai serait de 7,68 µM. Cette quantité d'ATP a permis la synthèse de 4 g/L de glucose-6P soit 15 000 µM, le module BL21-AckA aurait permis de dépasser 2000 fois la production possible à partir de l'ATP extracellulaire naturellement présent. Ces résultats montrent que le procédé de l'invention permet de valoriser un cofacteur à l'état de traces pour une bioproduction, ce qui particulièrement intéressant d'un point de vue économique.

### REFERENCES

Alvarez, C. L., G. Corradi, N. Lauri, I. Marginedas-Freixa, M. F. Leal Denis, N. Enrique, S. M. Mate, V. Milesi, M. A. Ostuni, V. Herlax, and P. J. Schwarzbaum. 2017. "Dynamic regulation of extracellular ATP in Escherichia coli." Biochem J 474 (8):1395-1416. doi: 10.1042/bcj20160879.
Brass, J. M, C. F. Higgins, M. Folley, P. A. Rugman, J. Birmingham, and P. B. Garland. 1986. Lateral diffusion of proteins in the periplasm of Escherichia coli. J. Bacteriol. 165:787-794.
Chung, C. T., and R. H. Miller. 1993. "Preparation and storage of competent Escherichia coli cells." Methods Enzymol 218:621-7. doi: 10.1016/0076-6879(93)18045-e.
Crans, Debbie C., and George M. Whitesides. 1983. "A convenient synthesis of disodium acetyl phosphate for use in in situ ATP cofactor regeneration." The Journal of Organic Chemistry 48 (18):3130-3132. doi: 10.1021/jo00166a048.
Dudek, Hanna M., Petra Popken, Edwin van Bloois, Wouter A. Duetz, and Marco W. Fraaije. 2013. "A Generic, Whole-Cell-Based Screening Method for Baeyer-Villiger Monooxygenases." Journal of Biomolecular Screening 18 (6):678-687. doi: 10.1177/1087057113480390.
Eichmann J, Oberpaul M, Weidner T, Gerlach D, Czermak P. Selection of High Producers From Combinatorial Libraries for the Production of Recombinant Proteins in Escherichia coli and Vibrio natriegens. Front Bioeng Biotechnol. 2019 Oct 4;7:254. doi: 10.3389/fbioe.2019.00254. PMID: 31637238; PMCID: PMC6788121.
Karyolaimos A, Ampah-Korsah H, Hillenaar T, Mestre Borras A, Dolata KM, Sievers S, Riedel K, Daniels R, de Gier JW. Enhancing recombinant protein yields in the E. coli periplasm by combining signal peptide and production rate screening. Front Microbiol. 2019;10:1511.
Mergulhao FJ, Monteiro GA. 2007. Periplasmic targeting of recombinant proteins in Escherichia coli. Methods in Molecular Biology 390:47-61.
Passariello, C., C. Forleo, V. Micheli, S. Schippa, R. Leone, S. Mangani, M. C. Thaller, and G. M. Rossolini. 2006. "Biochemical characterization of the class B acid phosphatase (AphA) of Escherichia coli MG1655." Biochim Biophys Acta 1764 (1):13-9. doi: 10.1016/j.bbapap.2005.08.028.
Popp, A., H. T. Nguyen, K. Boulahya, C. Bideaux, S. Alfenore, S. E. Guillouet, and E. Nevoigt. 2008. "Fermentative production of L-glycerol 3-phosphate utilizing a Saccharomyces cerevisiae strain with an engineered glycerol biosynthetic pathway." Biotechnol Bioeng 100 (3):497-505. doi: 10.1002/bit.21777.
Spari D, Beldi G. Extracellular ATP as an Inter-Kingdom Signaling Molecule: Release Mechanisms by Bacteria and Its Implication on the Host. Int J Mol Sci. 2020 Aug 421(15):5590. doi: 10.3390/ijms21155590. PMID: 32759857; PMCID: PMC7432876.
Stock, J. B., B. Rauch, and S. Roseman. 1977. Periplasmic space in Salmonella typhimurium and Escherichia coli. J. Biol. Chem. 252:7850-7861.
Wilks JC, Slonczewski JL. pH of the cytoplasm and periplasm of Escherichia coli: rapid measurement by green fluorescent protein fluorimetry. J Bacteriol. 2007 Aug;189(15):5601-7. doi: 10.1128/JB.00615-07. Epub 2007 Jun 1. PMID: 17545292; PMCID: PMC1951819.
Zhou Y, Wang L, Yang F, Lin X, Zhang S, Zhao ZK. Determining the extremes of the cellular NAD(H) level by using an Escherichia coli NAD(+)-auxotrophic mutant. Appl Environ Microbiol. 2011 Sep;77(17):6133-40. doi: 10.1128/AEM.00630-11. Epub 2011 Jul 8. PMID: 21742902; PMCID: PMC3165392.

## Revendications

1. Procédé de production d'un composé d'intérêt à partir d'un substrat organique, **caractérisé en ce qu'**il comprend les étapes consistant à :
a) - préparer un mélange réactionnel comprenant, dans un milieu aqueux :
- n bactéries, n étant un nombre entier au moins égal à 2, chaque bactérie étant génétiquement modifiée pour exprimer dans son espace périplasmique une seule enzyme E1, E2, ..., En, chacune desdites n bactéries exprimant une enzyme différente de celle exprimée dans les autres bactéries,
- ladite enzyme E1 étant apte à catalyser une première réaction R1 à partir dudit substrat organique pour fournir un premier produit P1, et optionnellement un premier coproduit CoP1,
- chacune desdites enzyme E2, ..., En, étant apte à catalyser une réaction R2, ..., Rn, à partir d'un produit ou d'un coproduit obtenu par une réaction Rn-1, pour fournir respectivement un produit P2, ..., Pn, et optionnellement un coproduit CoP2, ..., CoPn,
- ledit substrat organique, et
- optionnellement, un cofacteur CoF1 dudit substrat organique , un cofacteur CoFn desdits produits obtenus par une réaction Rn-1, , et/ou un substrat complémentaire SC d'un coproduit obtenu par une réaction Rn-1,
b) - laisser réagir le mélange réactionnel ainsi obtenu, et
c) - séparer la biomasse du surnageant et extraire de celui-ci ledit composé d'intérêt constitué par l'un des produits P1, P2, ..., Pn,
**caractérisé en ce que**
lesdites n bactéries génétiquement modifiées sont des bactéries didermes à Gram négatif, choisies chacune dans la famille des Enterobacteriaceae, des Alcaligenaceae, des Vibrionaceae, ou des Pseudomonadaceae, et
**en ce que** chacune desdites n bactéries est génétiquement modifiée pour exprimer un seul polypeptide, comprenant respectivement une desdites enzymes E1, E2, ..., En, liée à un peptide signal adressant ledit polypeptide dans l'espace périplasmique de ladite bactérie ou un peptide d'ancrage membranaire incluant ledit peptide signal.

2. Procédé selon la revendication 1, **caractérisé en ce que** le mélange réactionnel comprend un cofacteur CoF1 dudit substrat organique, une bactérie exprimant une enzyme E1 apte à catalyser une première réaction R1 à partir dudit substrat organique et du cofacteur CoF1 pour former un premier produit P1 et un premier coproduit CoP1 ; et une bactérie exprimant une deuxième enzyme E2 apte à catalyser une deuxième réaction R2 à partir du premier produit P1 pour former un deuxième produit P2, ou à partir du premier coproduit CoP1 pour former un deuxième coproduit CoP2, l'un des deux au moins étant consommé dans une troisième réaction, ou récupéré à l'étape c) en tant que composé d'intérêt.

3. Procédé selon la revendication précédente, **caractérisé en ce que** le mélange réactionnel comprend en outre au moins un substrat complémentaire SCn dudit au moins un cofacteur CoFn.

4. Procédé selon l'une quelconque des revendications précédentes, **caractérisé en ce que** ledit au moins un cofacteur CoFn est introduit en totalité ou en partie dans le mélange réactionnel à l'étape a), ou durant l'étape b).

5. Procédé selon l'une des revendications précédentes, **caractérisé en ce que** ledit au moins un cofacteur CoFn est formé au moins en partie dans le mélange réactionnel par une réaction Rn-1 catalysée par une enzyme En-1 apte à former un produit Pn-1 et un coproduit CoPn-1, ledit coproduit CoPn-1 étant identique au cofacteur CoFn.

6. Procédé selon la revendication précédente, **caractérisé en ce que** la quantité du premier cofacteur CoF1 introduite dans le mélange réactionnel à l'étape a) correspond à une concentration molaire au moins 20 fois inférieure à la concentration molaire initiale dudit substrat organique.

7. Procédé selon l'une quelconque des revendications précédentes, **caractérisé en ce que** le substrat organique de la première réaction est choisi parmi les hydrates de carbone, les aldéhydes, les alcools, les acides organiques, les carbamates, les hydrocarbures, les acides aminés, le dioxyde de carbone.

8. Procédé selon l'une des revendications précédentes, **caractérisé en ce que** les enzymes E1, E2, ..., En, sont chacune choisies parmi les kinases, les déshydrogénases, les phosphatases, les réductases, les isomérases, les transférases.

9. Procédé selon l'une des revendications précédentes, **caractérisé en ce que** l'une au moins des enzymes E1, E2, ..., En, catalyse une réaction fournissant un des coproduits CoP1, CoP2, ..., CoPn, choisi parmi l'ATP, l'ADP, l'AMP, l'UTP, l'UDP, l'UMP, le NAD+, le NADH, le NADP+, le NADPH, le FAD, le FADH2, le coenzyme A, ou catalyse une réaction utilisant l'un d'eux comme cofacteur.

10. Procédé selon l'une des revendications précédentes, **caractérisé en ce qu'**après l'étape c), la biomasse séparée du surnageant est réutilisée pour préparer un nouveau mélange réactionnel selon l'étape a), avec ou sans étape de conservation intermédiaire.

11. Procédé selon l'une des revendications précédentes, **caractérisé en ce que**, les étapes a) et b) sont réalisées en ajoutant dans le mélange réactionnel contenant lesdites n bactéries, en permanence ou à intervalle de temps,
- optionnellement, un cofacteur CoF1 dudit substrat organique, un cofacteur CoFn desdits produits obtenus par une réaction Rn-1,et/ou un substrat complémentaire SC d'un coproduit obtenu par une réaction Rn-1,
et en laissant réagir le mélange réactionnel ainsi obtenu en mode continu ou semicontinu.

12. Mélange réactionnel pour la production d'un composé d'intérêt à partir d'un substrat organique, **caractérisé en ce qu'**il contient, dans un milieu aqueux :
- n bactéries, n étant un nombre entier au moins égal à 2, chaque bactérie étant génétiquement modifiée pour exprimer dans son espace périplasmique une seule enzyme E1, E2, ..., En, chacune desdites n bactéries exprimant une enzyme différente de celle exprimée dans les autres bactéries,
- ladite enzyme E1 étant apte à catalyser une réaction R1 à partir dudit substrat organique pour fournir un produit P1, et optionnellement un coproduit CoP1,
- chacune desdites enzyme E2, ..., En, étant apte à catalyser une réaction R2, ..., Rn, à partir d'un produit ou d'un coproduit obtenu par une réaction Rn-1, pour fournir respectivement un produit P2, ..., Pn, et optionnellement un coproduit CoP2, ..., CoPn,
- ledit substrat organique, et
- optionnellement, un cofacteur CoF1 dudit substrat organique, un cofacteur CoFn desdits produits obtenus par une réaction Rn-1, , et/ou un substrat complémentaire SC d'un coproduit obtenu par une réaction Rn-1,
**caractérisé en ce que**
lesdites n bactéries génétiquement modifiées sont des bactéries didermes à Gram négatif, choisies chacune dans la famille des Enterobacteriaceae, des Alcaligenaceae, des Vibrionaceae, ou des Pseudomonadaceae, et
**en ce que** chacune desdites n bactéries est génétiquement modifiée pour exprimer un seul polypeptide, comprenant respectivement une desdites enzymes E1, E2, ..., En, liée à un peptide signal adressant ledit polypeptide dans l'espace périplasmique de ladite bactérie ou un peptide d'ancrage membranaire incluant ledit peptide signal.

13. Utilisation d'un mélange réactionnel selon la revendication 12 pour produire un composé d'intérêt choisi parmi les hydrates de carbone, les aldéhydes, les alcools, les acides organiques, les carbamates, les hydrocarbures, les acides aminés.

## Patentansprüche

1. Verfahren zur Herstellung einer Verbindung von Interesse aus einem organischen Substrat, **dadurch gekennzeichnet, dass** es die folgenden Schritte umfasst:
a)- Herstellen eines Reaktionsgemischs, das in einem wässrigen Medium umfasst:
- n Bakterien, wobei n eine Ganzzahl von mindestens 2 ist, wobei jedes Bakterium genetisch so verändert ist, dass es in seinem Periplasma ein einziges Enzym E1, E2, ..., En exprimiert, wobei jedes der n Bakterien ein Enzym exprimiert, das sich von dem in den anderen Bakterien exprimierten Enzym unterscheidet,
- wobei das Enzym E1 geeignet ist, eine erste Reaktion R1 ausgehend von dem organischen Substrat zu katalysieren, um ein erstes Produkt P1 und optional ein erstes Nebenprodukt CoP1 zu liefern,
- wobei jedes der Enzyme E2, ..., En geeignet ist, eine Reaktion R2, ..., Rn ausgehend von einem Produkt oder von einem Nebenprodukt, das durch eine Reaktion Rn-1 erhalten wurde, zu katalysieren, um jeweils ein Produkt P2, ..., Pn und optional ein Nebenprodukt CoP2, ..., CoPn zu liefern,
- das organische Substrat und
- optional, einen Cofaktor CoF1 des organischen Substrats, einen Cofaktor CoFn der durch eine Reaktion Rn-1 erhaltenen Produkte und/oder ein komplementäres Substrat SC eines durch eine Reaktion Rn-1 erhaltenen Nebenprodukts,
b)- Reagieren lassen des so erhaltenen Reaktionsgemisch, und
c)- Trennen der Biomasse vom Überstand und Extrahieren aus diesem die Verbindung von Interesse, bestehend aus einem der Produkte P1, P2, ..., Pn,
**dadurch gekennzeichnet, dass**
die n genetisch veränderten Bakterien gramnegative Didermen sind, die jeweils aus der Familie der Enterobacteriaceae, der Alcaligenaceae, der Vibrionaceae oder der Pseudomonadaceae ausgewählt sind, und
dadurch, dass jedes der n Bakterien genetisch so verändert ist, dass es ein einziges Polypeptid exprimiert, das jeweils eines der Enzyme E1, E2, ..., En umfasst, verbunden mit einem Signalpeptid, das das Polypeptid in den periplasmatischen Raum des Bakteriums leitet, oder einem Membranankerbpeptid, das das Signalpeptid enthält.

2. Verfahren nach Anspruch 1, **dadurch gekennzeichnet, dass** das Reaktionsgemisch einen Cofaktor CoF1 des organischen Substrats, ein Bakterium, das ein Enzym E1 exprimiert, das geeignet ist, eine erste Reaktion R1 ausgehend von dem organischen Substrat und von dem Cofaktor CoF1 zu katalysieren, um ein erstes Produkt P1 und ein erstes Nebenprodukt CoP1 zu bilden; und ein Bakterium, das ein zweites Enzym E2 exprimiert, das geeignet ist, eine zweite Reaktion R2 ausgehend von dem ersten Produkt P1 zur Bildung eines zweiten Produkts P2 oder ausgehend von dem ersten Nebenprodukt CoP1 zur Bildung eines zweiten Nebenprodukts CoP2 zu katalysieren, umfasst, wobei mindestens eines der beiden in einer dritten Reaktion verbraucht oder in Schritt c) als Verbindung von Interesse zurückgewonnen wird.

3. Verfahren nach vorstehendem Anspruch, **dadurch gekennzeichnet, dass** das Reaktionsgemisch ferner mindestens ein zu dem mindestens einen Cofaktor CoFn komplementäres Substrat SCn umfasst.

4. Verfahren nach einem der vorstehenden Ansprüche, **dadurch gekennzeichnet, dass** der mindestens eine Cofaktor CoFn ganz oder teilweise in Schritt a) oder während Schritt b) in das Reaktionsgemisch eingebracht wird.

5. Verfahren nach einem der vorstehenden Ansprüche, **dadurch gekennzeichnet, dass** der mindestens eine Cofaktor CoFn mindestens teilweise in dem Reaktionsgemisch durch eine Reaktion Rn-1 gebildet wird, die durch ein Enzym En-1 katalysiert wird, das geeignet ist, ein Produkt Pn-1 und ein Nebenprodukt CoPn-1 zu bilden, wobei das Nebenprodukt CoPn-1 mit dem Cofaktor CoFn identisch ist.

6. Verfahren nach vorstehendem Anspruch, **dadurch gekennzeichnet, dass** die Menge des ersten Cofaktors CoF1, die in Schritt a) in das Reaktionsgemisch eingebracht wird, einer molaren Konzentration entspricht, die mindestens 20-mal niedriger als die anfängliche molare Konzentration des organischen Substrats ist.

7. Verfahren nach einem der vorstehenden Ansprüche, **dadurch gekennzeichnet, dass** das organische Substrat der ersten Reaktion aus Kohlenhydraten, Aldehyden, Alkoholen, organischen Säuren, Carbamaten, Kohlenwasserstoffen, Aminosäuren, Kohlendioxid ausgewählt ist.

8. Verfahren nach einem der vorstehenden Ansprüche, **dadurch gekennzeichnet, dass** die Enzyme E1, E2, ..., En jeweils aus der Gruppe der Kinasen, der Dehydrogenasen, der Phosphatasen, der Reduktasen, der Isomerasen, der Transferasen ausgewählt sind.

9. Verfahren nach einem der vorstehenden Ansprüche, **dadurch gekennzeichnet, dass** mindestens eines der Enzyme E1, E2, ..., En eine Reaktion katalysiert, die eines der Nebenprodukte CoP1, CoP2, ..., CoPn liefert, die aus ATP, ADP, AMP, UTP, UDP, UMP, NAD+, NADH, NADP+, NADPH, FAD, FADH2, Coenzym A ausgewählt sind, oder eine Reaktion katalysiert, die eines davon als Cofaktor verwendet.

10. Verfahren nach einem der vorstehenden Ansprüche, **dadurch gekennzeichnet, dass** nach Schritt c) die vom Überstand getrennte Biomasse wiederverwendet wird , um ein neues Reaktionsgemisch gemäß Schritt a) herzustellen, mit oder ohne Zwischenspeicherungsschritt.

11. Verfahren nach einem der vorstehenden Ansprüche, **dadurch gekennzeichnet, dass** die Schritte a) und b) durchgeführt werden, indem in das die n Bakterien enthaltende Reaktionsgemisch kontinuierlich oder in Zeitintervallen hinzugefügt wird
- optional ein Cofaktor CoF1 des organischen Substrats, ein Cofaktor CoFn der durch eine Reaktion Rn-1 erhaltenen Produkte und/oder ein komplementäres Substrat SC eines durch eine Reaktion Rn-1 erhaltenen Nebenprodukts
und man das so erhaltene Reaktionsgemisch im kontinuierlichen oder halbkontinuierlichen Modus reagieren lässt.

12. Reaktionsgemisch zur Herstellung einer Verbindung von Interesse aus einem organischen Substrat, **dadurch gekennzeichnet, dass** es in einem wässrigen Medium enthält:
- n Bakterien, wobei n eine Ganzzahl von mindestens 2 ist, wobei jedes Bakterium genetisch so verändert ist, dass es in seinem Periplasma ein einziges Enzym E1, E2, ..., En exprimiert, wobei jedes der n Bakterien ein Enzym exprimiert, das sich von dem in den anderen Bakterien exprimierten Enzym unterscheidet,
- wobei das Enzym E1 geeignet ist, eine Reaktion R1 ausgehend von dem organischen Substrat zu katalysieren, um ein Produkt P1 und optional ein Nebenprodukt CoP1 zu liefern,
- wobei jedes der Enzyme E2, ..., En geeignet ist, eine Reaktion R2, ..., Rn ausgehend von einem Produkt oder von einem Nebenprodukt, das durch eine Reaktion Rn-1 erhalten wurde, zu katalysieren, um jeweils ein Produkt P2, ..., Pn und optional ein Nebenprodukt CoP2, ..., CoPn zu liefern,
- das organische Substrat, und
- optional, einen Cofaktor CoF1 des organischen Substrats, einen Cofaktor CoFn der durch eine Reaktion Rn-1 erhaltenen Produkte und/oder ein komplementäres Substrat SC eines durch eine Reaktion Rn-1 erhaltenen Nebenprodukts,
**dadurch gekennzeichnet, dass**
die n genetisch veränderten Bakterien gramnegative Didermen sind, die jeweils aus der Familie der Enterobacteriaceae, der Alcaligenaceae, der Vibrionaceae oder der Pseudomonadaceae ausgewählt sind, und
dadurch, dass jedes der n Bakterien genetisch so verändert ist, dass es ein einziges Polypeptid exprimiert, das jeweils eines der Enzyme E1, E2, ..., En umfasst, verbunden mit einem Signalpeptid, das das Polypeptid in den periplasmatischen Raum des Bakteriums leitet, oder einem Membranankerbpeptid, das das Signalpeptid enthält.

13. Verwendung eines Reaktionsgemischs nach Anspruch 12 zur Herstellung einer Verbindung von Interesse, ausgewählt aus Kohlenhydraten, Aldehyden, Alkoholen, organischen Säuren, Carbamaten, Kohlenwasserstoffen, Aminosäuren.

## Claims

1. A process for producing a compound of interest from an organic substrate, **characterized in that** it comprises the steps consisting of:
a) - preparing a reaction mixture comprising, in an aqueous medium,
- n bacteria, n being an integer at least equal to 2, each bacterium being genetically modified to express in its periplasmic space a single enzyme E1, E2, ..., En, each of said n bacteria expressing an enzyme different from the one expressed in the other bacteria,
- said enzyme E1 being able to catalyze a first reaction R1 from said organic substrate to provide a first product P1, and, optionally, a first coproduct CoP1,
- each of said enzymes E2, ..., En, being capable of catalyzing a reaction R2, ..., Rn, from a product or a coproduct obtained by a reaction Rn-1, to provide, respectively, a product P2, ..., Pn, and optionally a coproduct CoP2, ..., CoPn,
- said organic substrate, and
- optionally, a cofactor CoF1 of said organic substrate, a cofactor CoFn of said products obtained by a reaction Rn-1, and/or a complementary substrate SC of a coproduct obtained by a reaction Rn-1,
b) - allowing the reaction mixture thus obtained to react, and
c) - separating the biomass from the supernatant and extracting therefrom said compound of interest consisting of one of the products P1, P2, ..., Pn,
**characterized in that**
said n genetically modified bacteria are Gram-negative diderm bacteria, each chosen from the Enterobacteriaceae, Alcaligenaceae, Vibrionaceae, or Pseudomonadaceae family, and
**in that** each of the n bacteria is genetically modified to express a single polypeptide, comprising , respectively, one of said enzymes E1, E2,..., En, linked to a signal peptide addressing said polypeptide into the periplasmic space of said bacterium or to a membrane anchoring peptide including said signal peptide.

2. The process according to claim 1, **characterized in that** the reaction mixture comprises a cofactor CoF1 of said organic substrate, a bacterium expressing an enzyme E1 capable of catalyzing a first reaction R1 from said organic substrate and the cofactor CoF1 to form a first product P1 and a first coproduct CoP1; and a bacterium expressing a second enzyme E2 capable of catalyzing a second reaction R2 from the first product P1 to form a second product P2, or from the first coproduct CoP1 to form a second coproduct CoP2, at least one of the two being consumed in a third reaction, or recovered in step c) as a compound of interest.

3. The process according to the preceding claim, **characterized in that** the reaction mixture can also comprise at least one substrate SCn complementary to said at least one cofactor CoFn.

4. The process according to any one of the preceding claims, **characterized in that** said at least one cofactor CoFn can be introduced entirely or partially into the reaction mixture in step a), or during step b).

5. The process according to one of the preceding claims, **characterized in that** said at least one cofactor CoFn is formed at least in part in the reaction mixture by a reaction Rn-1 catalyzed by an enzyme En-1 capable of forming a product Pn-1 and a coproduct CoPn-1, said coproduct CoPn-1 being identical to the cofactor CoFn.

6. The process according to the preceding claim, **characterized in that** the quantity of the first cofactor CoF1 introduced into the reaction mixture in step a) corresponds to a molar concentration at least 20 times lower than the initial molar concentration of said organic substrate.

7. The process according to any one of the preceding claims, **characterized in that** the organic substrate of the first reaction is chosen from carbohydrates, aldehydes, alcohols, organic acids, carbamates, hydrocarbons, amino acids and carbon dioxide.

8. The process according to one of the preceding claims, **characterized in that** the enzymes E1, E2,..., En are each chosen from kinases, dehydrogenases, phosphatases, reductases, isomerases and transferases.

9. The process according to one of the preceding claims, **characterized in that** at least one of the enzymes E1, E2,..., En catalyzes a reaction providing one of the coproducts CoP1, CoP2,..., CoPn, chosen from ATP, ADP, AMP, UTP, UDP, UMP, NAD+, NADH, NADP+, NADPH, FAD, FADH₂, coenzyme A, or catalyzes a reaction using one of them as cofactor.

10. The process according to one of the preceding claims, **characterized in that** after step c) of the process that is the object of the present invention, the biomass separated from the supernatant is reused to prepare a new reaction mixture according to step a), with or without an intermediate preservation step.

11. The process according to one of the preceding claims, **characterized in that** steps a) and b) are carried out by adding to the reaction mixture containing said n bacteria, continuously or at intervals of time,
- optionally, a cofactor CoF1of said organic substrate, a cofactor CoFn of said products obtained by a reaction Rn-1, and/or a complementary substrate SC of a coproduct obtained by a reaction Rn-1, and by allowing the reaction mixture thus obtained to react continuously or semi-continuously.

12. A reaction mixture for the production of a compound of interest from an organic substrate, **characterized in that** it contains, in an aqueous medium:
- n bacteria, n being an integer at least equal to 2, each bacterium being genetically modified to express in its periplasmic space a single enzyme E1, E2,...En, each of said n bacteria expressing an enzyme different from the one expressed in the other bacteria,
- said enzyme E1 being able to catalyze a first reaction R1 from said organic substrate to provide a first product P1, and, optionally, a first coproduct CoP1,
- each of said enzymes E2, ..., En, being capable of catalyzing a reaction R2, ..., Rn, from a product or a coproduct obtained by a reaction Rn-1, to provide, respectively, a product P2, ..., Pn, and optionally a coproduct CoP2, ..., CoPn,
- said organic substrate, and
- optionally, a cofactor CoF1 of said organic substrate, a cofactor CoFn of said products obtained by a reaction Rn-1, and/or a complementary substrate SC of a coproduct obtained by a reaction Rn-1,
**characterized in that**
said n genetically modified bacteria are Gram-negative diderm bacteria, each chosen from the Enterobacteriaceae, Alcaligenaceae, Vibrionaceae, or Pseudomonadaceae family, and
**in that** each of the n bacteria is genetically modified to express a single polypeptide, comprising , respectively, one of said enzymes E1, E2,..., En, linked to a signal peptide addressing said polypeptide into the periplasmic space of said bacterium or to a membrane anchoring peptide including said signal peptide.

13. A use of a reaction mixture according to claim 12 for producing a compound of interest chosen from carbohydrates, aldehydes, alcohols, organic acids, carbamates, hydrocarbons and amino acids.
